# EUROPEAN PATENT APPLICATION

(11) **EP 4 368 184 A2**
(43) Date of publication of application: **15.05.2024**
(21) Application number: 24166495.2
(22) Date of filing: 19.11.2020
(51) Int. Cl.: A61K 31/506

(54) **NOVEL COMPOUNDS FOR THE DIAGNOSIS, TREATMENT AND PREVENTION OF DISEASES ASSOCIATED WITH THE AGGREGATION OF ALPHA-SYNUCLEIN**

(30) Priority: 19.11.2019 EP 19210073
(62) Divisional of application: 20810960.3
(71) Applicant: Modag GmbH, 55234 Wendelsheim (DE); Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a compound represented by the general formula Ila or IIb 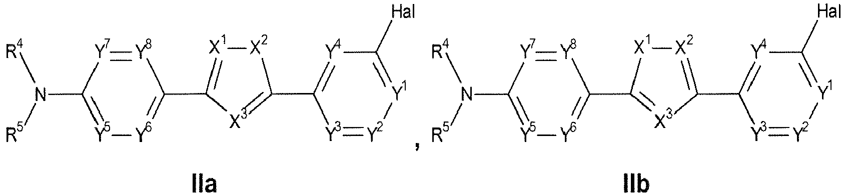 or a solvate or salt thereof, wherein 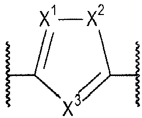 is selected from 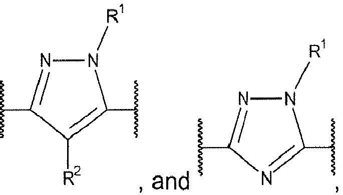 and 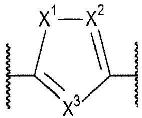 is selected from 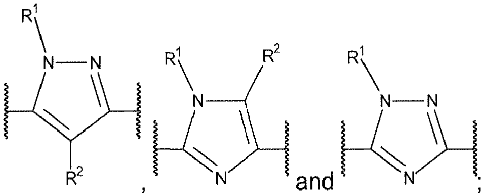 **Y¹**, **Y²**, **Y³**, **Y⁴**, **Y⁵**, **Y⁶**, **Y⁷** and **Y⁸** are independently selected from CR³ and N, with the proviso that at least one of Y¹, Y², Y³, Y⁴, Y⁵, Y⁶, Y⁷ and Y⁸ is N and with the proviso that in the formulae la or Ib, at least one of Y¹, Y³, Y⁴, and Y⁶ is N; **R¹** is independently selected from hydrogen, C₁₋₄ alkyl and -(CH₂)-O-P(=O)(OR)(OR), wherein C₁₋₄ alkyl can be optionally substituted by one or more halogen; **R²** is independently selected from hydrogen, halogen, and C₁₋₄ alkyl, wherein C₁₋₄ alkyl can be optionally substituted by one or more halogen; **R** is hydrogen or a cation; **R³** is hydrogen; **R⁴** and **R⁵** are independently selected from H and C₁₋₄ alkyl, wherein C₁₋₄ alkyl can be optionally substituted by one or more halogen or wherein R⁴ and R⁵ together with the nitrogen atom to which they are bound form a piperazinyl ring which is substituted by one or more R⁶; and **R⁶** is independently selected from halogen, C₁₋₄ alkyl, OH and C₁₋₄ alkoxy, wherein C₁₋₄ alkyl and C₁₋₄ alkoxy can be optionally substituted by one or more halogen; and **Hal** is halogen.

## Description

### SUMMARY OF THE INVENTION

The present invention refers to novel compounds which are suitable for imaging alpha-synuclein and for diagnosing diseases which are associated with the aggregation of alpha-synuclein. The compounds are also useful for treating and preventing diseases which are associated with the aggregation of alpha-synuclein.

### BACKGROUND OF THE INVENTION

A large number of neurological and neurodegenerative diseases are known, many of which are presently not curable and difficult to diagnose. All common neurodegenerative diseases are characterized by the misfolding, aggregation, and deposition of specific proteins in the brain. These diseases include medical conditions such as Parkinson's disease (PD), Dementia with Lewy Bodies (DLB), Multiple System Atrophy (MSA), Alzheimer's disease, progressive supranuclear palsy, corticobasal degeneration, frontotemporal dementia, Creutzfeldt-Jakob disease and many others.

Synucleinopathies are a group of diseases characterized by accumulation and deposition of aggregated and misfolded alpha-synuclein protein (αSYN). Synucleinopathies include Parkinson's disease (PD), Dementia with Lewy Bodies (DLB), and Multiple System Atrophy (MSA). These diseases differ in the distribution of the accumulation and deposition of aggregated and misfolded alpha-synuclein protein in the central and peripheral nervous system. Neuropathologically, they can be distinguished from other neurodegenerative diseases by the disease-specific accumulation and deposition of aggregated and misfolded alpha-synuclein protein, whereas other neurodegenerative diseases are characterized by the accumulation and deposition of other aggregated and misfolded proteins. For example, Alzheimer's disease is characterized by aggregated and misfolded Abeta (Aβ) and tau protein, progressive supranuclear palsy and corticobasal degeneration are characterized by aggregated and misfolded tau protein, also some cases of frontotemporal dementia are characterized by aggregated and misfolded tau protein. Creutzfeldt-Jakob disease is characterized by aggregated and misfolded prion protein.

The disease-specific accumulation and deposition of aggregated and misfolded proteins provides a target both for therapy as well as for diagnosis by compounds which bind to these aggregated and misfolded protein deposits.

One option for diagnostic detection of disease-specific accumulation and deposition of aggregated and misfolded proteins is the use of detectably labeled compounds that show specific and selective high-affinity binding to said deposits of aggregated protein. This can be done, for example, by using compounds that are labeled with suitable radioactive isotopes and by using PET imaging for detection. Whereas compounds are available for clinical use for the PET imaging of Abeta and tau, no compounds have been invented so far that can be used for diagnostic PET imaging of alpha-synuclein deposits in synucleinopathies (Kotzbauer, P.T., Tu, Z. and Mach, R.H., Current status of the development of PET radiotracers for imaging alpha synuclein aggregates in Lewy bodies and Lewy neurites. Clin. Transl. Imaging, 2017. 5: p. 3-14). Compounds developed and tested by other groups so far lacked a suitable combination of properties including high binding affinity to aggregated and misfolded alpha-synuclein, sufficient selectivity in binding affinity compared to aggregated and misfolded other proteins, especially Abeta and tau (required for differential diagnosis of the different diseases characterized by disease-specific accumulation and deposition of aggregated and misfolded proteins and for the ability to accurately detect the presence of aggregated and misfolded alpha-synuclein also in patients that have more than one disease at the same time, i.e. Dementia with Lewy Bodies and Alzheimer's disease). Moreover, the required properties include the ability to pass the blood-brain barrier and bind to intracellular deposits, low unspecific binding to brain tissue, and a rapid wash out of non-bound compound from the brain.

WO 2009/146343 refers to certain pyrazoles, 1,2,4-oxadiazoles, and 1,3,4-oxadiazoles which are tracers in positron emission tomography (PET) imaging to study amyloid deposits in brain *in vivo* to allow diagnosis of Alzheimer's disease. Alzheimer's disease is characterized by the aggregation of Abeta and tau proteins and the mentioned PET tracers bind to aggregates of these proteins. In contrast, the present invention targets diseases characterized by aggregation of alpha-synuclein. For selective diagnostic imaging of aggregated alpha-synuclein a selective binding to alpha-synuclein and no to low binding to aggregated Abeta and tau is required.

WO 00/66578 describes specific NPY antagonists that are useful for the treatment of NPY mediated disease/conditions such as obesity. It is mentioned that in addition to the "direct" effect of the compounds of WO0066578 on the NPY5 subtype, there are diseases/conditions that will benefit from the weight loss such as insulin resistance, impaired glucose tolerance, type II diabetes, hypertension, hyperlipidemia, cardiovascular disease, gall stones, certain cancers, sleep apnea, etc.

WO 2010/000372 relates to a specific compound which is useful in the treatment or prevention of diseases linked to protein aggregation and/or neurodegenerative diseases. The compounds of WO 2010/000372 are particularly suitable for treating diseases linked to protein aggregation including Parkinson's disease. They have been shown to bind to several different aggregated proteins including Abeta and tau. Therefore, they may be used to diagnose disorders linked to protein aggregation but it is not possible to reliably distinguish between disorders which are linked to alpha-synuclein aggregation and other disorders which are linked to amyloid protein aggregation such as tauopathies or Alzheimer's disease. This, however, would be important because the clinical manifestations of disorders linked to protein aggregation are very similar and it would be very desirable to distinguish, e.g., between the various disorders in order to adapt the treatment accordingly. Moreover, the molecules described in WO 2010/000372 have a high unspecific binding to lipids and hydrophobic proteins which results in strong unspecific binding in brain tissue and other tissues. Thus, they do not reach the required specific binding and signal-to-noise ratio to alpha-synuclein as required for a PET tracer.

In view of the foregoing, there was a need for compounds which have improved properties as a diagnostic. In particular, the specificity of binding to alpha-synuclein should be improved. The unspecific binding in brain tissue and other tissues should be reduced, the binding affinity should be increased, and the physiological half-life should be reduced.

US 2005/0075375 discloses specific heterocyclic compounds for treating hepatitis C virus.

### BRIEF DESCRIPTION OF THE FIGURE

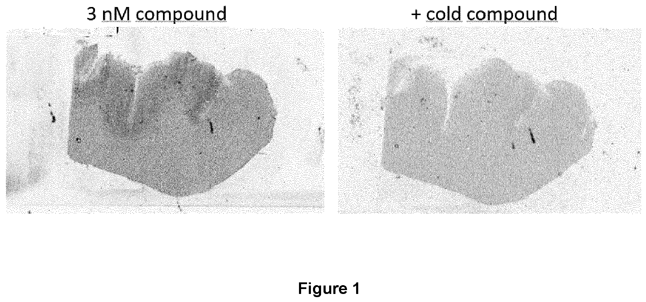

Figure 1: Autoradiography using human brain tissue from a patient suffering from dementia with Lewy bodies using [3H]-compound 1

### DETAILED DESCRIPTION OF THE INVENTION

The present invention relates to a compound represented by the general formula Ia, Ib, Ila or IIb 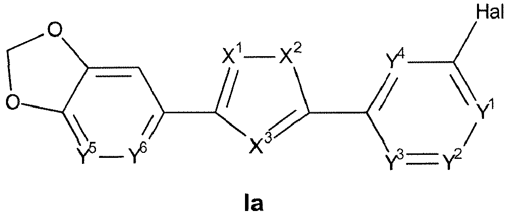 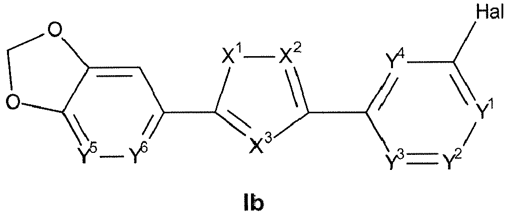 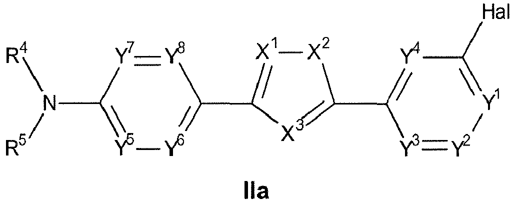 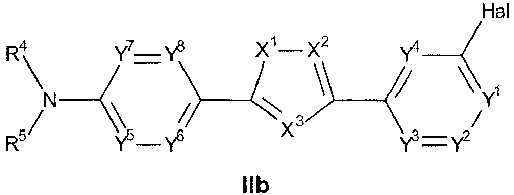 X¹, X², and X³ are independently selected from CR², N and NR¹, with the proviso that at least two of X¹, X², and X³ are N or NR¹. It is understood that N and NR¹ are present as valency permits, i.e., N can only be present at a position =X¹-, =X²- or =X³- and NR¹ can only be present at a position -X¹- or -X²-.

Examples of 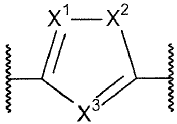 include

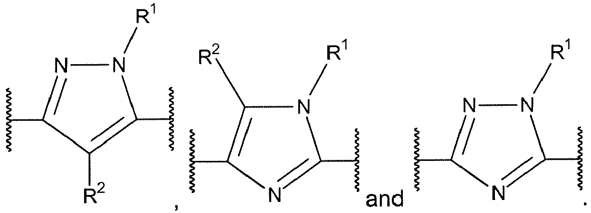

Examples of 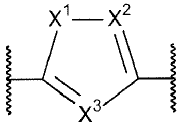 include

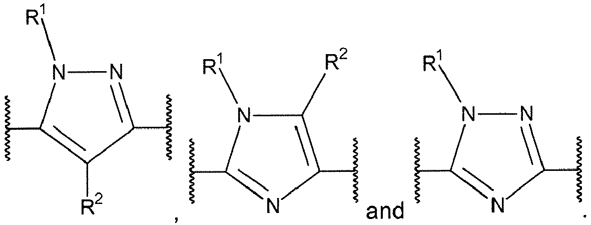

In a preferred embodiment, 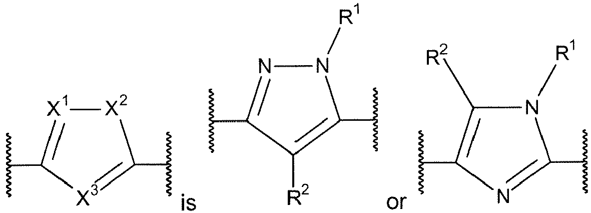 (more preferably 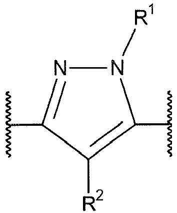 ) or 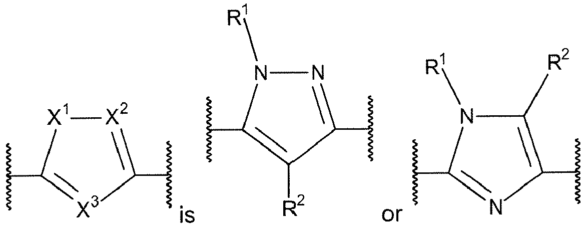 (more preferably 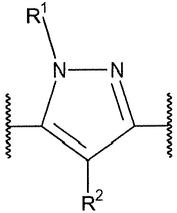 ).

**Y¹, Y², Y³, Y⁴, Y⁵, Y⁶, Y⁷** and **Y⁸** are independently selected from CR³ and N, with the proviso that at least one of Y¹, Y², Y³, Y⁴, Y⁵, Y⁶, Y⁷ and Y⁸ is N.

In a preferred embodiment in the formulae Ia or Ib, Y¹, Y², Y³, Y⁴, Y⁵, and Y⁶ are independently selected from CR³ and N, with the proviso that at least one of Y¹, Y², Y³, Y⁴, Y⁵, and Y⁶ is N. In a more preferred embodiment, one or two or three of Y¹, Y², Y³, Y⁴, Y⁵, and Y⁶ is N, even more preferably one or two of Y¹, Y², Y³, Y⁴, Y⁵, and Y⁶ is N, yet more preferably one of Y¹, Y², Y³, Y⁴, Y⁵, and Y⁶ is N. In a preferred embodiment, Y¹ is N.

In a preferred embodiment in the formulae Ia or Ib, at least one of Y¹, Y³, Y⁴, and Y⁶ is N. In a further preferred embodiment in the formulae Ia or Ib, Y¹ is N and at least one of Y³, Y⁴, and Y⁶ is N.

In a preferred embodiment in the formulae Ia or Ib, at least one of Y¹, Y³, Y⁴, and Y⁶ is N and the others of Y¹, Y², Y³, Y⁴, Y⁵, and Y⁶ are CR³ (such as CH). In a further preferred embodiment in the formulae Ia or Ib, Y¹ is N, at least one of Y³, Y⁴, and Y⁶ is N and the others of Y², Y³, Y⁴, Y⁵, and Y⁶ are CR³ (such as CH).

In a preferred embodiment in the formulae Ia or Ib, Y¹ is N and the others of Y², Y³, Y⁴, Y⁵, and Y⁶ are CR³, more preferably Y¹ is N and Y², Y³, Y⁴, Y⁵, and Y⁶ are CH.

In a preferred embodiment in the formulae Ila or IIb, Y¹, Y², Y³, Y⁴, Y⁵, Y⁶, Y⁷ and Y⁸ are independently selected from CR³ and N, with the proviso that at least one of Y¹, Y², Y³, Y⁴, Y⁵, Y⁶, Y¹ and Y⁸ is N. In a more preferred embodiment, one or two or three of Y¹, Y², Y³, Y⁴, Y⁵, Y⁶, Y¹ and Y⁸ is N, even more preferably one or two of Y¹, Y², Y³, Y⁴, Y⁵, Y⁶, Y⁷ and Y⁸ is N, yet more preferably one of Y¹, Y², Y³, Y⁴, Y⁵, Y⁶, Y⁷ and Y⁸ is N. In a preferred embodiment, Y¹ is N.

In a preferred embodiment in the formulae Ila or lib, at least one of Y¹, Y³, Y⁴, Y⁵, Y⁶, Y⁷ and Y⁸ is N. In a preferred embodiment in the formulae Ila or IIb, at least one of Y¹, Y³, Y⁴, Y⁵, and Y⁷ is N. In a further preferred embodiment in the formulae Ila or lib, Y¹ is N and at least one of Y³, Y⁴, Y⁵, Y⁶, Y⁷ and Y⁸ is N.

In a preferred embodiment in the formulae Ila or IIb, at least one of Y¹, Y³, Y⁴, Y⁵, Y⁶, Y⁷ and Y⁸ is N and the others of Y¹, Y², Y³, Y⁴, Y⁵, Y⁶, Y⁷ and Y⁸ are CR³ (such as CH). In a preferred embodiment in the formulae Ila or IIb, at least one of Y¹, Y³, Y⁴, Y⁵, and Y⁷ is N and the others of Y¹, Y², Y³, Y⁴, Y⁵, Y⁶, Y⁷ and Y⁸ are CR³ (such as CH). In a further preferred embodiment in the formulae Ila or IIb, Y¹ is N, at least one of Y³, Y⁴, Y⁵, Y⁶, Y⁷ and Y⁸ is N and the others of Y², Y³, Y⁴, Y⁵, Y⁶, Y⁷ and Y⁸ are CR³ (such as CH). In a preferred embodiment in the formulae Ila or IIb, Y¹ is N, at least one of Y³, Y⁴, Y⁵, and Y⁷ is N and the others of Y², Y³, Y⁴, Y⁵, Y⁶, Y⁷ and Y⁸ are CR³ (such as CH).

In a preferred embodiment in the formulae Ila or IIb, Y' is N and the others of Y², Y³, Y⁴, Y⁵, Y⁶, Y⁷ and Y⁸ are CR³, more preferably Y¹ is N and Y², Y³, Y⁴, Y⁵, Y⁶, Y⁷ and Y⁸ are CH.

It has been surprisingly found that the introduction of one or more nitrogen atoms as Y¹, Y², Y⁶, Y⁴, Y⁵, Y⁶, Y⁷ and Y⁸ strongly increases the selectivity of the binding to alpha-synuclein, so that corresponding disorders which are linked to alpha-synuclein aggregation such as Parkinson's disease, Dementia with Lewy Bodies, and Multiple System Atrophy can be distinguished from disorders with aggregation of other proteins such as Alzheimer's disease where predominantly Abeta and tau aggregate. Furthermore, the signal-to-noise ratio is improved.

R¹ is selected from hydrogen, C₁₋₄ alkyl and -(CH₂)-O-P(=O)(OR)(OR), wherein C₁₋₄ alkyl can be optionally substituted by one or more halogen. In a preferred embodiment, R¹ is hydrogen, methyl or 2-fluoroethyl. If present, it is preferred that -(CH₂)-O-P(=O)(OR)(OR) is attached to X¹ or X².

R is hydrogen or a cation. The cation can be any cation that is pharmaceutically acceptable. Preferably, the cation is a monovalent cation. Examples are sodium, lithium, potassium, ammonium and protonated forms of ethanolamine, choline, lysine, meglumine, piperazine, and tromethamine. Preferably, the cation is sodium. In the compounds of the present invention both R can be hydrogen, both R can be cations (the same or different cations), or one R can be hydrogen and the other one can be a cation. Preferably, both R are sodium. Bivalent cations such as Ca²⁺, Mg²⁺, and Zn²⁺ or trivalent cations such as Al³⁺ are possible but not preferred, as the resulting salts are less water-soluble. Compounds in which R¹ is -(CH₂)-O-P(=O)(OR)(OR) and their synthesis are described in WO 2017/102893, which is incorporated herein by reference.

R² is independently selected from hydrogen, halogen, and C₁₋₄ alkyl, wherein C₁₋₄ alkyl can be optionally substituted by one or more halogen. In a preferred embodiment, R² is hydrogen.

R³ is hydrogen, halogen, C₁₋₄ alkyl, OH and C₁₋₄ alkoxy, wherein C₁₋₄ alkyl and C₁₋₄ alkoxy can be optionally substituted by one or more halogen. In a preferred embodiment, R³ is hydrogen or fluorine. Even more preferably R³ is hydrogen.

R⁴ and R⁵ are independently selected from H and C₁₋₄ alkyl, wherein C₁₋₄ alkyl can be optionally substituted by one or more halogen or wherein R⁴ and R⁵ together with the nitrogen atom to which they are bound form a 4- to 6-membered saturated, heterocyclic ring which optionally contains one or more heteroatoms selected from O and N in addition to the nitrogen atom to which R⁴ and R⁵ are bound, wherein the 4- to 6-membered saturated, heterocyclic ring can be optionally substituted by one or more R⁶.

In one embodiment, R⁴ and R⁵ are independently selected from H and C₁₋₄ alkyl, wherein C₁₋₄ alkyl can be optionally substituted by one or more halogen. Preferably, R⁴ and R⁵ are independently selected from H and C₁₋₄ alkyl. In a preferred embodiment, at least one of R⁴ and R⁵ is C₁₋₄ alkyl, wherein C₁₋₄ alkyl can be optionally substituted by one or more halogen. In a more preferred embodiment, R⁴ is hydrogen and R⁵ is C₁₋₄ alkyl, wherein C₁₋₄ alkyl can be optionally substituted by one or more halogen. In a more preferred embodiment, at least one of R⁴ and R⁵ is C₁₋₄ alkyl. In a more preferred embodiment, R⁴ is hydrogen and R⁵ is C₁₋₄ alkyl.

In a further embodiment, R⁴ and R⁵ together with the nitrogen atom to which they are bound form a 4- to 6-membered saturated, heterocyclic ring which optionally contains one or more (e.g., one) heteroatoms selected from O and N in addition to the nitrogen atom to which R⁴ and R⁵ are bound, wherein the 4- to 6-membered saturated, heterocyclic ring can be optionally substituted by one or more R⁶. In a preferred embodiment, the 4- to 6-membered saturated, heterocyclic ring is selected from azetidinyl, pyrrolidinyl, piperidinyl, morpholinyl and piperazinyl, wherein azetidinyl, pyrrolidinyl, piperidinyl, morpholinyl and piperazinyl can be optionally substituted by one or more R⁶. More preferably, the 4- to 6-membered saturated, heterocyclic ring is selected from azetidinyl, pyrrolidinyl, piperidinyl, morpholinyl and piperazinyl, wherein the N-atom of piperazinyl can be optionally substituted by R⁶.

R⁶ is independently selected from halogen, C₁₋₄ alkyl, OH and C₁₋₄ alkoxy, wherein C₁₋₄ alkyl and C₁₋₄ alkoxy can be optionally substituted by one or more halogen, preferably R⁶ is C₁₋₄ alkyl or fluorine.

Hal is halogen, such as Br, Cl and F, preferably Hal is Br or F.

m is the number of R³ groups other than hydrogen in the ring which contains Y¹, Y², Y³, and Y⁴. m is an integer of 0 to mₘₐₓ, with mₘₐₓ being the number of carbon atoms in the ring which contains Y¹, Y², Y³, and Y⁴. Preferably, m is 0.

n is the number of R³ groups other than hydrogen in the ring which contains Y⁵, Y⁶, Y⁷, and Y⁸ (formula IIa and IIb). n is an integer of 0 to nₘₐₓ, with nₘₐₓ being the number of carbon atoms in the ring which contains Y⁵, Y⁶, Y⁷, and Y³. Preferably, n is 0.

p is the number of R³ groups other than hydrogen in the ring which contains Y⁵ and Y⁶ (formula Ia and Ib). p is an integer of 0 to pₘₐₓ, with pₘₐₓ being the number of carbon atoms in the ring which contains Y⁵ and Y⁶. Preferably, p is 0.

The compounds of the present invention can also be present in the form of prodrugs, solvates or salts thereof.

The compounds of the present invention form salts which are also within the scope of this invention. Reference to a compound of the present invention herein is understood to include reference to salts thereof, unless otherwise indicated. Pharmaceutically acceptable (i.e., non-toxic, physiologically acceptable) salts are preferred, although other salts are also useful, e.g., in isolation or purification steps which may be employed during preparation or in *in vitro* methods. Salts of the compounds of the present invention may be formed, for example, by reacting a compound with an amount of acid, such as an equivalent amount, in a medium such as one in which the salt precipitates or in an aqueous medium followed by lyophilization.

The compounds which contain a basic moiety may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (e.g., 2-hydroxyethanesulfonates), lactates, maleates, methanesulfonates, naphthalenesulfonates (e.g., 2-naphthalenesulfonates), nicotinates, nitrates, oxalates, pectinates, persulfates, phenylpropionates (e.g., 3-phenylpropionates), phosphates, picrates, pivalates, propionates, salicylates, succinates, sulfates (e.g., those formed with sulfuric acid), sulfonates (e.g., those mentioned herein), tartrates, thiocyanates, toluenesulfonates such as tosylates, undecanoates, and the like.

Prodrugs and solvates of the compounds of the invention are also contemplated herein. The term "prodrug" as employed herein denotes a compound which, upon administration to a subject, undergoes chemical conversion by metabolic or chemical processes to yield a compound of the present invention or a salt and/or solvate thereof.

Solvates of the compounds of the present invention include, for example, hydrates.

All stereoisomers of the present compounds (e.g., those which may exist due to asymmetric carbons on various substituents), including enantiomeric forms and diastereomeric forms, are contemplated within the scope of this invention. Individual stereoisomers of the compounds of the invention may, for example, be substantially free of other isomers (e.g., as a pure or substantially pure optical isomer having a specified activity), or may be admixed, for example, as racemates or with all other, or other selected, stereoisomers. The chiral centers of the compounds of the present invention may have the S- or R-configuration as defined by the IUPAC 1974 Recommendations.

The racemic forms can be resolved by physical methods, such as fractional crystallization, separation or crystallization of diastereomeric derivatives or separation by chiral column chromatography. The individual optical isomers can be obtained from the racemates by any suitable method, including without limitation, salt formation with an optically active acid followed by crystallization.

All configurational isomers of the compounds of the present invention are contemplated, either in admixture or in pure or substantially pure form. The definition of compounds of the present invention embraces both cis (Z) and trans (E) alkene isomers, as well as cis and trans isomers of cyclic hydrocarbon or heterocyclic rings.

Deuterated versions of the claimed compounds can also be provided. The position at which the deuteration is present is not particularly limited but it can, for instance, be in the C₁₋₄ alkyl of R⁴ and R⁵.

Throughout the specification, groups and substituents thereof may be chosen to provide stable moieties and compounds.

The compounds of the present invention can be provided in the form of a diagnostic or pharmaceutical composition which optionally includes a pharmaceutically acceptable carrier or excipient.

In accordance with the present invention, the term "diagnostic composition" relates to a composition for the determination of the presence of aggregated alpha-synuclein underlying a disease linked to alpha-synuclein aggregation.

In a preferred embodiment, the compound of the present invention is detectable or detectably labeled. It is understood in accordance with the present invention that a compound is detectable or detectably labeled if its presence can be monitored by conventional techniques such as NMR spectroscopy, single photon emission computed tomography (SPECT), optical detection, positron emission tomography (PET), electron microscopy, magnetic resonance imaging (MRI), spectrometry, chromatography, ELISA assay, detection of radioactive emission, preferably by PET, scintillation counting or gamma counting, more preferably by PET.

When the compounds of the present invention are to be used as probes for imaging aggregated alpha-synuclein, they should be labeled. The specific nature of the label will depend on the method which is to be used for imaging. Typically radioactive labels which emit positrons (PET) and which have a short half-life such as ¹⁸F, ¹¹C, ¹²⁵I, ¹²³I, ¹³¹I, ⁷⁷Br and ⁷⁶Br, in particular ¹⁸F and ¹¹C, will be useful. Due to their short half-lives, the labeled compounds of the present invention should be prepared shortly before they are used for testing. Consequently, the diagnostic composition of the present invention can also be provided in the form of a kit, which consists of at least two precursors of a compound of the present invention, which are reacted to form the desired compound of the present invention.

A skilled person will be able to devise methods with which the detectable label can be attached to the compound of the present invention. The following schemes can be served as illustrative examples.

### Labeling with ¹⁸F:

2-[¹⁸F]Fluoroethyl tosylate is a useful precursor for incorporating ¹⁸F via fluoroethylation of compounds containing nitrogen, oxygen and sulfur nucleophiles as described in WO2010/000372, page 32, Scheme A the for preparation of compound 21 and compound 22 starting from compound 23. The compounds **A** and **B** can be prepared in the same manner. Another method includes a direct nucleophilic replacement of a suitable leaving group as shown for the transformation of **C** to **D** (cf. J. Med. Chem. 2013, 56, 4568-4579, scheme 2) or the transformation of compound 24 to compound 12 in Scheme 1.

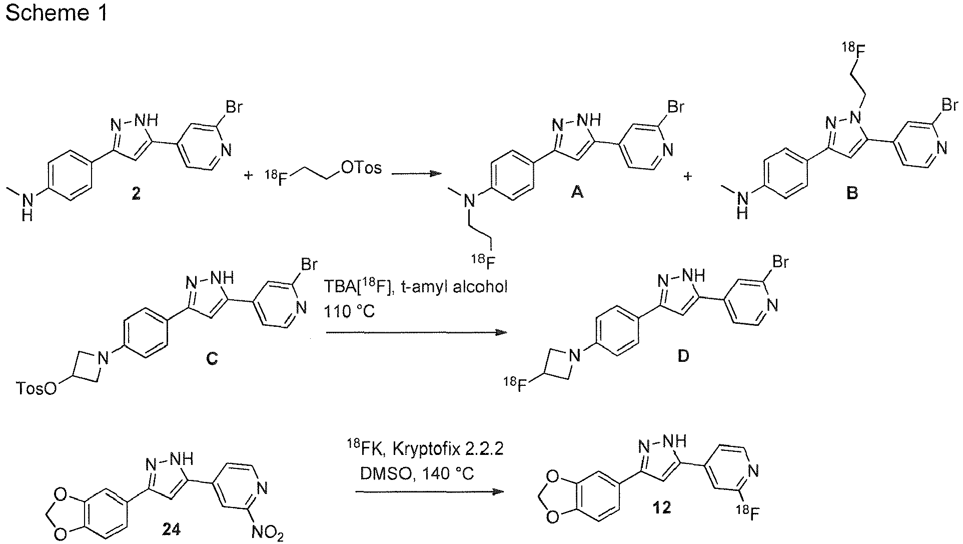

### Labeling with ¹¹C:

The compounds of invention labeled with ¹¹C can be prepared by direct nucleophilic alkylation of the suitable precursor with [¹¹C] Mel as described in WO 2010/000372, page 32, Scheme B for the preparation of compound 27 and compound 28 starting from compound 23. The compound 1 of the present invention can be synthesized analogously starting from compound 2. In the same manner, the compound 18 and compound 19 can be synthesized starting from compound 9 as shown in scheme 2. Alternatively, a simple and accessible method (J.M. Hooker et al., Angew. Chem. Int. Ed. 2008, 47, 5989-5992) for the production of [¹¹C]formaldehyde as a solution in dimethylformamide by converting [¹¹C]methyl iodide to [¹¹C]formaldehyde under mild conditions with no loss of specific activity can be used for the transformation of compound 2 to compound 1 via reductive amination.

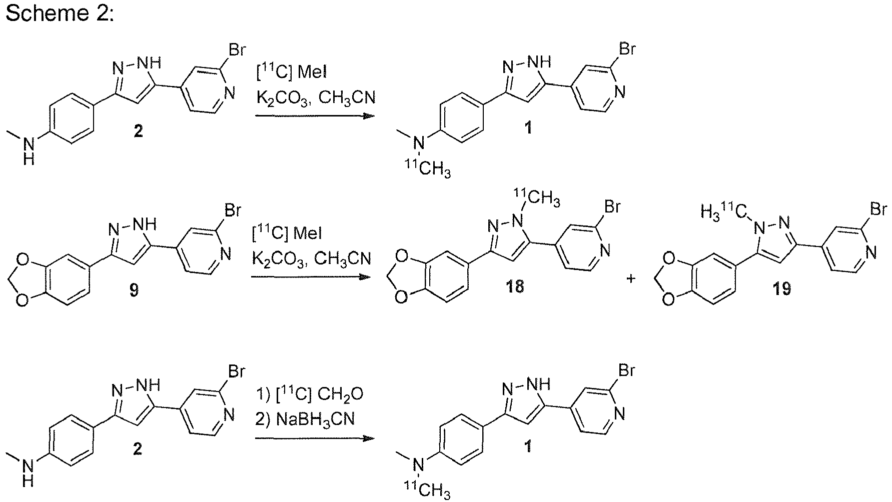

The present invention provides a method of imaging deposits of aggregated alpha-synuclein, which comprises the steps of:
(i) introducing a detectable quantity of a composition comprising a detectably labeled compound of the present invention into a subject;
(ii) allowing sufficient time for the compound to be associated with the aggregated alpha-synuclein; and
(iii) detecting the compound associated with the aggregated alpha-synuclein.

The composition comprising the detectably labeled compound may be introduced into the subject by any route of administration described below, such as for example orally or parenterally. The labeled compound can be introduced into a patient and after a time span sufficient for the compound to become associated with the aggregated alpha-synuclein, the labeled compound is detected noninvasively inside the patient. Alternatively, the labeled compound can be introduced into a patient, sufficient time is allowed for the compound to become associated with the aggregated alpha-synuclein, and then a sample of tissue from the patient is taken and the labeled compound is detected in the tissue apart from the patient. A tissue sample can also be removed from a patient before introducing the labeled compound into the tissue sample. After allowing for a sufficient amount of time for the compound to become bound to the aggregated alpha-synuclein, the compound can be detected.

The imaging of aggregated alpha-synuclein can also be carried out quantitatively so that the amount of aggregated alpha-synuclein can be determined.

The present invention further relates to the compound of the present invention as well as a prodrug, solvate or salt thereof for the use in the treatment or prevention of a disease linked to the aggregation of alpha-synuclein.

Further embodiments are the use of a compound of the present invention for the preparation of a pharmaceutical composition for treating or preventing a disease linked to the aggregation of alpha-synuclein as well as a method of treating or preventing a disease linked to the aggregation of alpha-synuclein comprising administering a therapeutically effective amount of a compound of the present invention to a patient in need thereof. This includes the application of the compound as a "pharmaceutical composition" as described below.

The term "aggregation", in accordance with the present invention, refers to the formation of oligomeric or multimeric complexes of alpha-synuclein, which may be accompanied by the integration of additional biomolecules, like carbohydrates, nucleic acids, lipids and/or metal ions, into the complexes.

The term "disease linked to aggregation of alpha-synuclein", as used herein, refers to those diseases which are characterized by the presence of aggregated alpha-synuclein. Such aggregated alpha-synuclein may form deposits in specific tissue, more preferably in nerve tissue or tissue of the brain. The extent of aggregation depends on the particular disease.

In accordance with the present invention, the term "pharmaceutical composition" relates to a composition for administration to a patient, preferably a mammal, more preferably a human patient. The pharmaceutical composition of the invention comprises the compounds recited above and optionally further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier or excipient. Examples of suitable pharmaceutical carriers and excipients are well-known in the art and include phosphate buffered saline solutions, water, emulsions, such as oil/water emulsions, various types of wetting agents, sterile solutions, organic solvents including DMSO etc. Compositions comprising such carriers can be formulated by well-known conventional methods.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations. The skilled person knows that the effective amount of pharmaceutical compositions administered to an individual will, inter alia, depend on the nature of the compound.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray. Preferably, they will be administered intravenously when used as a PET tracer for diagnosis and orally when used for treating or preventing a disease.

By "pharmaceutically acceptable carrier" is meant a non-toxic solid, semisolid or liquid filler, diluent, encapsulating material or formulation auxiliary of any type.

The term "parenteral" as used herein refers to modes of administration which include intravenous, intramuscular, intraperitoneal, intrasternal, subcutaneous and intraarticular injection and infusion.

For therapeutic purposes, the pharmaceutical composition can also be suitably administered by sustained release systems. Suitable examples of sustained-release compositions include semi-permeable polymer matrices in the form of shaped articles, e.g., films, or microcapsules. Sustained-release matrices include polylactides (U.S. Pat. No. 3,773,919, EP 58 481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133 988). Sustained release pharmaceutical compositions can also include liposomally entrapped compounds. Liposomes containing the pharmaceutical composition are prepared by methods known per se: DE 32 18 121; Epstein et al., Proc. Natl. Acad. Sci. (USA) 82:3688-3692 (1985); Hwang et al., Proc. Natl. Acad. Sci. (USA) 77:4030-4034 (1980); EP 52 322; EP 36 676; EP 88 046; EP 143 949; EP 142 641; Japanese Pat. Appl. 83-118008; U.S. Pat. Nos. 4,485,045 and 4,544,545; and EP 102324. Ordinarily, the liposomes are of the small (about 200 - 800 Angstroms) unilamellar type in which the lipid content is greater than about 30 mol% cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably, the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 µm membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-injection.

The present invention further relates to a method of treating or preventing a disease linked to alpha-synuclein aggregation comprising administering a therapeutically effective amount of a compound of the present invention to a patient in need thereof.

As used herein, the term "therapeutically effective amount" refers to an amount sufficient to elicit the desired biological response. In the present invention, the desired biological response is the inhibition of alpha-synuclein aggregation and/or the reduction in the amount of aggregated alpha-synuclein present in the tissue.

The present invention also relates to the use of a compound as defined above for inhibiting alpha-synuclein aggregation *in vitro* or *ex vivo.*

The disease linked to alpha-synuclein aggregation is not particularly limited and is typically selected from Parkinson's disease, dementia with Lewy bodies, and multiple system atrophy.

The following examples are intended to illustrate the invention. However, they are not to be construed as limiting.

### EXAMPLES

### Example 1: Synthesis and testing

### Chemical synthetic procedures

The following methods are presented with details as to the preparation of compounds of the invention and the illustrative examples. A compound of the invention can be prepared from known or commercially available starting materials and reagents by one skilled in the art of organic synthesis.

All starting materials and solvents were of commercial grade and were used as received unless noted otherwise.

**Thin layer chromatography (TLC)** was conducted using Macherey-Nagel precoated sheets, 0.25 mm ALUGRAM^{®} SIL G/UV254 plates, detection with UV and/or by charring with 10 wt% ethanolic phosphomolybdic acid reagent followed by heating at 200 °C.

**Flash column chromatography** was performed using Merck silica gel 60 (0.063-0.100 mm).

**Analytical high performance liquid chromatography (HPLC)** was performed by using a Waters HPLC system with a Waters 996 Photodiode Array Detector. All separations involved a mobile phase of 0.1% trifluoroacetic acid (TFA) (v/v) in water and 0.1% TFA in acetonitrile. HPLC was performed using a reversed-phase (RP) column Eurospher RP 18, 100 Å, 5 µm, 250×4.6 mm at flow rate of 1 mL·min⁻¹.

**Electrospray ionization mass spectrometry (ESI-MS)** and **liquid chromatography** / **mass spectrometry (LC/MS) analyses** were obtained by using a Waters Micromass ZQ 4000 mass spectrometer in conjunction with the Waters HPLC apparatus described above.

**NMR spectra** were recorded using a 400 MHz Bruker Avance spectrometer (Bruker AG, Rheinstetten, Germany) equipped with a TXI HCN z-gradient probe. All spectra were processed using TOPSPIN 3.1 (Bruker AG, Karlsruhe, Germany). ¹H NMR chemical shifts (δ) are reported in parts per million (ppm) relative to CHCl₃, DMSO-d5 and TFA-d1 as internal standards. Data are reported as follows: chemical shift, multiplicity (s = singlet, d = doublet, t = triplet, q = quartet, qi = quintet, dd = doublet of doublets, dt = doublet of triplets, b = broadened, m = multiplet), coupling constants (J, given in Hz), integration. ¹³C NMR chemical shifts (δ) are reported in parts per million (ppm) relative to CDCl₃, DMSO-d6 and TFA-d1 as internal standards. The following experiments were used to record the resonances of the compounds: ¹H-1D, ¹³C-1D NMR spectra and ¹³C-APT (attached proton test with a single J-evolution time of 1/145 s, spectra were processed such that quaternary and methylene groups have a positive sign and methyl and methine groups have a negative sign). To resolve overlap of resonances and recover undetectable resonances in ¹H and APT spectra, 2D-[¹³C,¹H]-HSQC (heteronuclear single quantum coherence), 2D-[¹³C,¹H]-HMBC (heteronuclear multiple bond correlation) and 2D-NOESY were recorded for some compounds.

Selected compounds (compound 1, compound 2, compound 12, compound 18) were tritiated for binding assays by RC TRITEC AG, Teufen, Switzerland using HIT exchange labeling with 99% tritium gas / Kerr's catalyst. The compounds were delivered as ethanolic solutions with 185 MBq packaging, 37 MBq/mL concentration and specific activity in the range 1.1 to 2.3 GBq/mmol.

### Method A: synthesis of 1H-pyrazoles

Those skilled in the art will recognize that the compound 11 A and compound 11 B depicted below are two tautomeric forms of the same compound. All such tautomeric forms are considered as part of this invention. As an illustration, all tautomeric forms of the pyrazole moiety as depicted, for example, for the compound 11 below are included in this invention. In a related manner, those skilled in the art will recognize that the compound names contained herein are based on a nomenclature convention wherein the tautomer configuration is depicted as with respect to compound 11 A below. Thus, the 1,3-benzodioxol substituent is in the three position. An alternative nomenclature convention would be based on the tautomer compound 11 B below and in that convention the 1,3-benzodioxol substituent is in the five position.

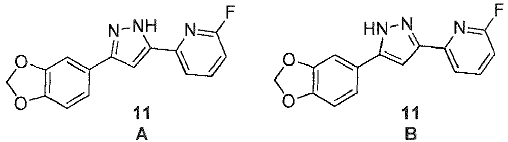

### Illustrative example: 2-[3-(1,3-Benzodioxol-5-yl)-1H-pyrazol-5-yl]-6-fluoropyridine compound 11

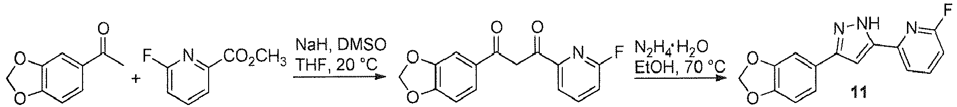

Sodium hydride (FW 24.00, 60% in oil, 3.9 mmol, 156 mg) was added to a solution of 1-(1,3-benzodioxol-5-yl)ethanone (FW 164.16, 492 mg, 3.00 mmol) and methyl 6-fluoropyridine-2-carboxylate (FW 155.13, 605 mg, 3.9 mmol) in DMSO (7.5 mL) and THF (1.9 mL), and the reaction mixture was stirred at 20 °C for 15 h. The reaction mixture was poured into 60 mL of ice and water containing AcOH (450 µL). The mixture was stirred for 1 h. The resultant precipitate was filtered off, washed with water (10 mL), hexane : EtOH = 5 : 1 (10 mL), hexane (10 mL), and dried in air to obtain a crude intermediate 1-(1,3-benzodioxol-5-yl)-3-(6-fluoropyridin-2-yl)propane-1,3-dione (594 mg) as a yellow solid. To a suspension of this crude intermediate in EtOH (20 mL), hydrazine hydrate (146 µL, 150 mg, 3 mmol) was added. The reaction mixture was stirred at 70 °C for 5 h, cooled down and concentrated *in vacuo.* The residue was suspended in MeOH (10 mL), boiled with stirring for 5 min, cooled down, filtered off, washed with MeOH (10 mL) and dried in high vacuum at 20 °C for 15 h to afford the pure product compound 11 (471 mg, 1.66 mmol, 55% over two steps) as a white solid.

### Method B: Synthesis of 1H-pyrazoles

### Illustrative example: 4-[3-(1,3-Benzodioxol-5-yl)-1H-pyrazol-5-yl]-2-bromopyridine compound 9

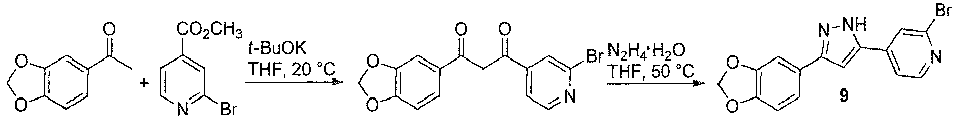

A solution of potassium *tert*-butoxide (FW 112.21, 281 mg, 2.5 mmol) in dry THF (5 mL) was added under nitrogen to a stirred solution of 1-(1,3-benzodioxol-5-yl)ethanone (FW 164.16, 328 mg, 2 mmol) and methyl 2-bromopyridine-4-carboxylate (FW 216.03, 518 mg, 2.4 mmol) in dry THF (5 mL). The reaction mixture was stirred for 15 h at 20 °C. A 20 µL aliquot was sampled, quenched with 1M phosphate buffer pH 7, extracted with EtOAc and analyzed by TLC. Full conversion of ketone was observed. The mixture was poured into 1M phosphate buffer pH 7 (15 mL) and ice water (15 mL), and stirred at 0 °C for 30 min. The resulting yellow solid was filtered off, washed with water (5×10 mL) and air-dried to give 656 mg (1.88 mmol, 94%) of crude intermediate 1-(1,3-benzodioxol-5-yl)-3-(2-bromopyridin-4-yl)propane-1.3-dione which was used for the next step without purification. A mixture of this intermediate and hydrazine monohydrate (FW 50.06, d 1.03; 274 µL, 282 mg, 5.64 mmol) in THF (10 mL) was stirred at 50 °C for 15 h. The cooled mixture was poured into water (40 mL) and stirred at 0 °C for 30 min. The resulting precipitate was filtered off, washed with water and air-dried. The crude product was crystallized from n-BuOH (10 mL) and DMF (1 mL) to give a pure product compound 9 (458 mg, 1.33 mmol, 67% over 2 steps) as a white powder.

### Method C: Removing of Boc protective group

### Illustrative example: 4-[5-(2-Bromopyridin-4-yl)-1H-pyrazol-3-yl]aniline compound 7

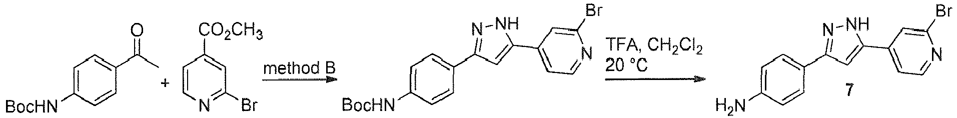

Trifluoroacetic acid (2 mL, 2.96 g, 26 mmol) was added to a suspension of crude tert-butyl {4-[5-(2-bromopyridin-4-yl)-1*H*-pyrazol-3-yl]phenyl}carbamate (FW 415.28, 865 mg, 2.08 mmol) prepared from *tert*-butyl *N*-(4-acetylphenyl)carbamate and methyl 2-bromopyridine-4-carboxylate according to Method B. The mixture was stirred at room temperature for 15 h and concentrated *in vacuo.* 1M phosphate buffer pH 7 (20 mL) was added, the resultant precipitate was filtered off, washed with water (2×10 mL) and air-dried to give 543 mg (1.72 mmol, 69% over 3 steps) of the desired product as a yellow-orange solid.

### Method D: Synthesis of 1H-pyrazoles

### Illustrative example: 4-[3-(1,3-Benzodioxol-5-yl)-1H-pyrazol-5-yl]-2-fluoropyridine compound 12

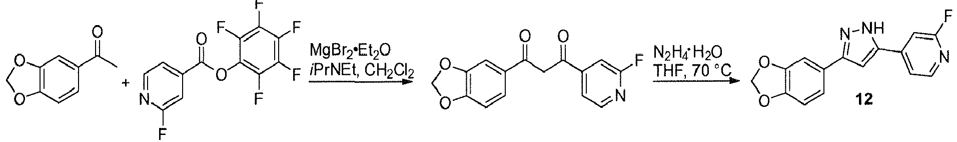

*i*Pr₂NEt (1.79 mL, 1.33 g, 10.26 mmol) was added to a stirred mixture of 1-(1,3-benzodioxol-5-yl)ethanone (561 mg, 3.42 mmol) and MgBr₂·Et₂O (1.56 g, 6.04 mmol) in CH₂Cl₂ (35 mL). The resultant suspension was stirred for 5 min, and then pentafluorophenyl 2-fluoropyridine-4-carboxylate (1.37 g, 4.45 mmol) in CH₂Cl₂ (7 mL) was added dropwise. The reaction mixture was stirred for 48 h. 1N Aqueous HCl (20 mL) was then added and stirring was continued for 5 min. The aqueous layer was extracted with CH₂Cl₂ (20 mL) and the combined organic extracts were dried (MgSO₄) and concentrated *in vacuo.* The residue was triturated with Et₂O (10 mL) and filtered. The solid was washed with Et₂O and air-dried to give the crude product (1.14 g, orange solid). The crude product was recrystallized from EtOAc (8 mL) and hexane (4 mL) to afford the pure intermediate 1-(1,3-benzodioxol-5-yl)-3-(2-fluoropyridin-4-yl)propane-1,3-dione (900 mg, 3.13 mmol, 92%) as an orange solid. This intermediate was suspended in THF (20 mL) and hydrazine monohydrate (292 µL, 300 mg, 6 mmol) was added. The reaction mixture was stirred at 70 °C for 5 h, cooled down and concentrated *in vacuo.* The residue was suspended in Et₂O (10 mL), boiled with stirring for 5 min, cooled down, filtered off, washed with Et₂O (2×10 mL) and dried in high vacuum at 20 °C for 15 h to afford the product compound 12 (722 mg, 2.55 mmol, 76% over 2 steps) as a white solid.

### Method E: Synthesis of pentafluorophenyl esters

### Illustrative example: Pentafluorophenyl 2-fluoropyridine-4-carboxylate compound 59

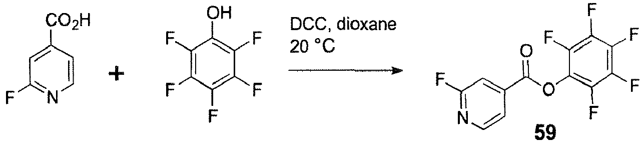

DCC (FW 206.33, 2.27 g, 11 mmol) was added to a stirred suspension of 2-fluoropyridine-4-carboxylic acid (1.41 g, 10 mmol) and pentafluorophenol (1.84 g, 10 mmol) in 1,4-dioxane (40 mL). Stirring was continued for 15 h, by which time a colorless precipitate had formed. The mixture was filtered through Celite^{®} and evaporated to give a semisolid. Chromatography over silica gel gave the ester compound 59 (2.21 g, 7.2 mmol, 72%) as a pure, colorless oil.

### Method F: Synthesis of 1H-pyrazoles

### Illustrative example: 6-[3-(3-Bromophenyl)-1H-pyrazol-5-yl]-1,3-dioxolo[4,5-c]pyridine compound 20

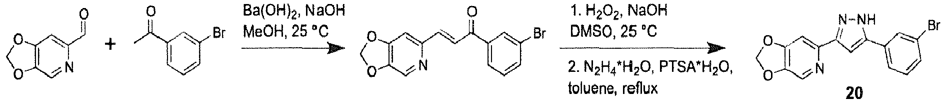

### Step 1

To a suspension of 1,3-dioxolo[4,5-c]pyridine-6-carboxaldehyde (WO/2019/208509) (35 mg, 0.23 mmol) and 1-(3-bromophenyl)ethanone (46 mg, 0.23 mmol) in methanol (0.7 mL) Ba(OH)₂*8H₂O (5 mg) and NaOH (0.5 mg) were added and the resulting mixture was stirred at room temperature (RT) overnight. After evaporation of methanol *in vacuo* the residue was triturated in water (5 mL), the solid was collected by filtration, washed with cold methanol (0.5 mL) and dried yielding an intermediate compound 1-(3-bromophenyl)-3-([1,3]dioxolo[4,5-c]pyridin-6-yl)prop-2-en-1-one (67 mg, 88%) as a white solid.

### Step 2

To a vigorously stirred suspension of 1-(3-bromophenyl)-3-([1,3]dioxolo[4,5-c]pyridin-6-yl)prop-2-en-1-one (67 mg, 0.2 mmol) in DMSO (0.8 mL) an aqueous solution of H₂O₂ (30%, 45 mg, 0.4 mmol) was added followed by dropwise addition of aqueous NaOH (10%, 16 µL, 0.04 mmol). The yellow mixture was stirred at RT for 1.5 h and poured into cold phosphate buffer (20 mL, 0.1M, pH 7). The oily precipitate was extracted by ethyl acetate (2*15 mL), combined organic fraction were dried over Na₂SO₄, concentrated *in vacuo* and the residue was resuspended in toluene (0.8 mL). The suspension in toluene was treated with hydrazine hydrate (35 mg, 0.7 mmol) and PTSA hydrate (5 mg) and the mixture was stirred under reflux for 1.5 h. After cooling down, phosphate buffer (0.3M, 20 mL) was added and the product was extracted with ethyl acetate (2*20 mL). The combined organic fractions were washed with brine (5 mL), dried over Na₂SO₄ and concentrated *in vacuo.* The crude residue was purified by column chromatography (15 g, silica gel 63-100, CHCl₃/MeOH = 100/1) to afford a yellow solid which was washed with Et₂O (1 mL) to provide compound 20 (25 mg, 36%) as a white solid.

### Method G: Synthesis of 1H-pyrazoles

### Illustrative example: 4-[4-(1,3-Benzodioxol-5-yl)-1H-imidazol-2-yl]-2-bromopyridine compound 48

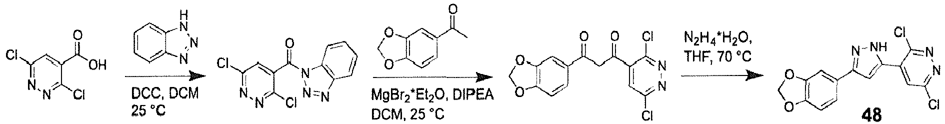

A mixture of 1-(1,3-benzodioxol-5-yl)ethanone (138 mg, 0.84 mmol), MgBr₂·Et₂O (542 mg, 2.1 mmol) in DCM (5 mL) was treated with DIPEA (323 mg, 426 µL, 2.5 mmol) and stirred at RT for 10 minutes. Next, the crude mixture of 1*H*-benzotriazol-1-yl(3,6-dichloropyridazin-4-yl)methanone, which was prepared separately by stirring of 3,6-dichloropyridazine-4-carboxylic acid (203 mg, 1.05 mmol), benzotriazole (125 mg, 1.05 mmol) and DCC (216 mg, 1.05 mmol) in dry DCM (5 mL) at 25 °C for 3 h, was added dropwise over 5 minutes. The resulting mixture was stirred at 25 °C for 12h, then treated with aqueous 0.5 M HCl (2 mL) and stirred for another 10 minutes at 25 °C. After addition of water (20 mL), the mixture was extracted with DCM (2*15 mL). Combined organic fractions were washed with brine, dried over Na₂SO₄ and concentrated *in vacuo.* The crude product was purified by column chromatography to provide intermediate 1-(1,3-benzodioxol-5-yl)-3-(3,6-dichloropyridazin-4-yl)propane-1,3-dione (190 mg, 67%) as an orange solid which was used in the next step without further purification. This intermediate was suspended in THF (4 mL) and hydrazine monohydrate (40 mg, 0.8 mmol) was added. The reaction mixture was stirred at 40 °C overnight, cooled down and concentrated *in vacuo.* The crude product was purified by column chromatography (silica gel 63-100, 20 g, chloroform/methanol = 100/1) to provide compound 48 (100 mg, 36% over two steps) as a light-yellow solid.

### Method H: Synthesis of 1H-imidazoles

### Illustrative example: 4-[4-(1,3-Benzodioxol-5-yl)-1H-imidazol-2-yl]-2-bromopyridine compound 33

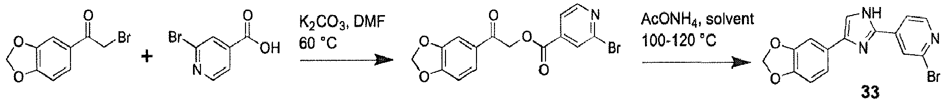

### Step 1

A suspension of 1-(1,3-benzodioxol-5-yl)-2-bromoethanone (729 mg, 3 mmol), 2-bromopyridine-4-carboxylic acid (606 mg, 3 mmol) and K₂CO₃ (414 mg, 3 mmol) in DMF (6 mL) was stirred at 55 to 60 °C for 6 h. After cooling down, the mixture was poured into water (60 mL), stirred for 10 minutes and the resulting precipitate was collected by filtration, washed with water (20 mL) on the filter and dried to provide an intermediate compound 2-(1,3-benzodioxol-5-yl)-2-oxoethyl 2-bromopyridine-4-carboxylate (899 mg, 87%) which was used in the next step without further purification.

### Step 2

A suspension of intermediate compound 2-(1,3-benzodioxol-5-yl)-2-oxoethyl 2-bromopyridine-4-carboxylate (364 mg, 1 mmol) and AcONH₄ (924 mg, 12 mmol) in toluene (7 mL) was heated at 100 °C with intensive stirring for 4 h. After cooling down the reaction mixture was treated with phosphate buffer (50 mL, 0.25 M, pH 7) and extracted with ethyl acetate (2*50 mL). Combined organic fractions were washed with brine, dried over Na₂SO₄ and concentrated *in vacuo.* The residue was purified by column chromatography (silica gel 63-100, 30 g, CHCl₃/MeOH = 100/1 → 100/3). The purified product was recrystallized from aqueous ethanol (90%) to provide compound 33 (140 mg, 41%) as a pale solid.

### Method I: Synthesis of 1H-1,2,4-triazoles

### Illustrative example 4-[3-(1,3-Benzodioxol-5-yl)-1H-1,2,4-triazol-5-yl]-2-bromopyridine Compound 30

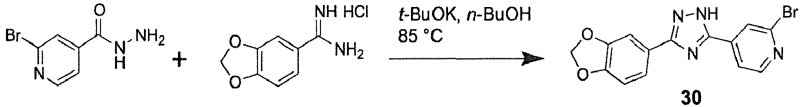

To a solution of *t*-BuOK (84 mg, 0.75 mmol) in n-BuOH (2 mL) benzo[1,3]dioxole-5-carboxamidine hydrochloride (100 mg, 0.5 mmol) was added at 0 °C and the mixture was stirred at RT for 20 minutes. Following the addition of 2-bromoisonicotinic acid hydrazide (108 mg, 0.5 mmol), a yellow suspension was stirred at 85 °C for 3h. After cooling to room temperature, ethanol (4 mL) was added and CO₂ was bubbled for 5 minutes into the suspension. After removing of the solvents *in vacuo* the residue was triturated in water (10 mL), the resulting precipitate was collected by filtration, washed with water (5 mL) and dried to provide compound 30 (85 mg, 49%) as a grey solid.

### Method J: Synthesis of 1H-1,2,4-triazoles

### Illustrative example 4-[3-(2-Bromopyridin-4-yl)-1H-1,2,4-triazol-5-yl]-N,N-dimethylaniline compound 44

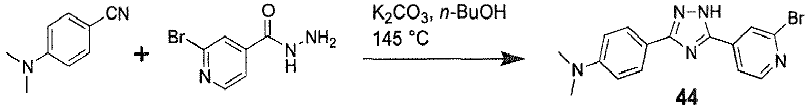

A mixture of 2-bromoisonicotinic acid hydrazide (151 mg, 0.7 mmol), 4-(dimethylamino)benzonitrile (307 mg, 2.1 mmol) and K₂CO₃ (48 mg, 0.5 mmol) in n-BuOH (2 mL) was stirred at 145 °C for 8h. The mixture was concentrated *in vacuo* and the crude product was purified by two column chromatography (silica gel 63-100, 20 g, CHCl₃/MeOH=100/1) and (silica gel 63-100, 20 g, acetone/hexane = 1/5) to provide compound 44 (20 mg, 8%) as a beige solid.

### Method K: Synthesis of 4-chloro-1H-pyrazoles

### 4-[3-(1,3-Benzodioxol-5-yl)-4-chloro-1H-pyrazol-5-yl]-2-bromopyridine, compound 28

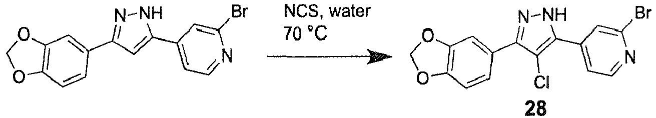

The suspension of compound 9 (100 mg, 0.29 mmol) and NCS (80 mg, 0.6 mmol) in water (3 mL) was stirred at 70 °C for 16 h, TLC showed the consumption of starting material. After cooling to RT, the precipitate was collected by filtration, washed with water (2*5 mL) and recrystallized from ethanol (8 mL) to provide compound 28 (52 mg, 47%) as a gray solid.

### Method L: Synthesis of 1-[4-(4-R-piperazin-1-yl)phenyl]ethanones

### Illustrative example 1-{4-[4-(2-Methoxyethyl)piperazin-1-yl]phenyl}ethenone

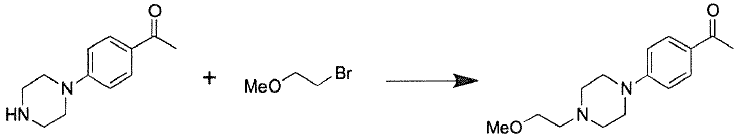

A mixture of 1-[4-(piperazin-1-yl)phenyl]ethanone (408 mg, 2 mmol), 1-bromo-2-methoxyethane (417 mg, 3 mmol), and Cs₂CO₃ (1304 mg, 4 mmol) in DMF (5 mL) was stirred at 25 °C overnight. After evaporation of DMF *in vacuo,* the residue was partitioned between water (25 mL) and ethyl acetate (35 mL), the aqueous phase was extracted with ethyl acetate (25 mL) and combined organic fractions were washed with brine, dried over Na₂SO₄ and concentrated *in vacuo.* The crude product was purified by column chromatography (30 g silica gel 63-100, chloroform → chloroform/MeOH = 100/2) to provide 1-{4-[4-(2-methoxyethyl)piperazin-1-yl]phenyl}ethenone (525 mg, 99%) as a light-yellow solid.
2-Methoxyethyl 2,6-dibromopyridine-4-carboxylate and 2-methoxyethyl 2,6-dichloropyridine-4-carboxylate were prepared according to a published protocol (Journal of Medicinal Chemistry (2012), 55, 10564-10571)
*tert*-Butyl (4-acetylphenyl)methylcarbamate, *tert*-butyl (4-acetylphenyl)-*N-*(²H₃)methylcarbamate, *tert*-butyl (4-acetylphenyl)ethylcarbamate and *tert-*butyl (4-acetylphenyl)(2-fluoroethyl)carbamate were prepared according to a published protocol (Organic Letters (2020), 22, 5522-5527).
1-[4-(4-Fluoropiperidin-1-yl)phenyl]ethanone and 1-[4-(3-fluoroazetidin-1-yl)phenyl]ethanone were prepared according to a published protocol (WO 2011071570 A1).

Pentafluorophenyl 2-fluoropyridine-4-carboxylate and pentafluorophenyl 2-chloropyridine-4-carboxylate were prepared according to method E. 1-{4-[4-(2-Methoxyethyl)piperazin-1-yl]phenyl}ethenone and 1-{4-[4-(2-fluoroethyl)piperazin-1-yl]phenyl}ethenone were prepared according to method L.

Exemplary compounds of the present invention are shown in Tables 1 and 2. Table 1 shows the name, structure, IUPAC name, starting materials for the preparation, method of synthesis and the chemical yield for a particular example compound. Table 2 shows the High Performance Liquid Chromatography (HPLC) retention time, molecular weight found using Low Resolution Mass Spectrometry coupled with HPLC, and proton Nuclear Magnetic Resonance (¹H-NMR) for a particular example compound.

**Table 1:**

| **No.** | **Structure** | **IUPAC** | **Reagents** | **Mtd./ Yield (%)** |
|---|---|---|---|---|
| 1 | 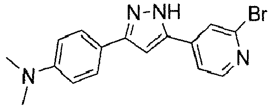 | 4-[3-(4-dimethylaminophenyl)-1*H*-pyrazol-5-yl]-2-bromopyridine | 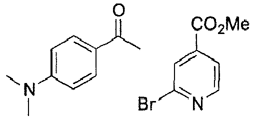 | A/69 |
| 2 | 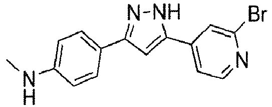 | 4-[5-(2-bromopyridin-4-yl)-1*H*-pyrazol-3-yl]-*N-*methylaniline | 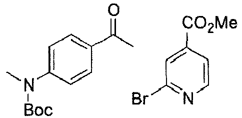 | B,C/47 |
| 3 | 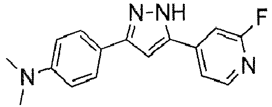 | 4-[5-(2-fluoropyridin-4-yl)-1*H*-pyrazol-3-yl]-*N*,*N*-dimethylaniline | 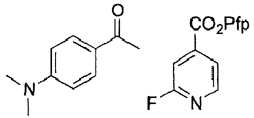 | D/53 |
| 4 | 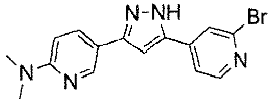 | 5-[5-(2-bromopyridin-4-yl)-1*H*-pyrazol-3-yl]-*N*,*N*-dimethylpyridin-2-amine | 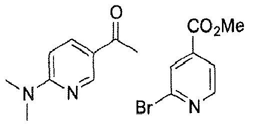 | A/40 |
| 5 | 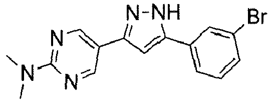 | 5-[5-(3-bromophenyl)-1*H*-pyrazol-3-yl]-*N*,*N-*dimethylpyrimidin-2-amine | 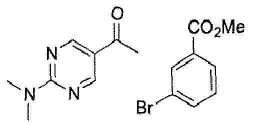 | A/60 |
| 6 | 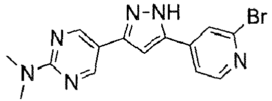 | 5-[5-(2-bromopyridin-4-yl)-1*H*-pyrazol-3-yl]-*N*,*N*-dimethylpyrimidin-2-amine | 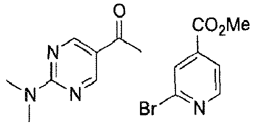 | A/40 |
| 7 | 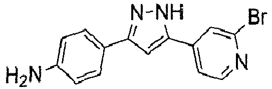 | 4-[5-(2-bromopyridin-4-yl)-1*H*-pyrazol-3-yl)aniline | 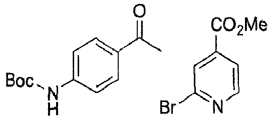 | B,C/69 |
| 8 | 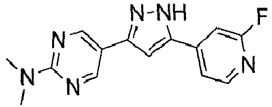 | 5-[5-(2-fluoropyridin-4-yl)-1*H*-pyrazol-3-yl]-*N*,*N*-dimethylpyrimidin-2-amine | 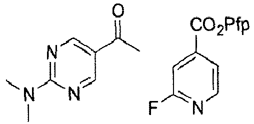 | D/69 |
| 9 | 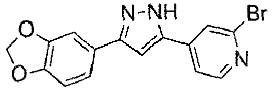 | 4-[3-(1,3-benzodioxol-5-yl)-1*H*-pyrazol-5-yl]-2-bromopyridine | 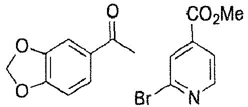 | B/67 |
| 10 | 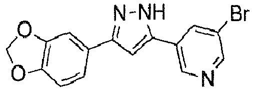 | 2-[3-(1,3-benzodioxol-5-yl)-1*H*-pyrazol-5-yl]-5-bromopyridine | 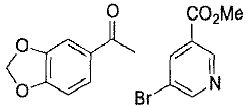 | B/79 |
| 11 | 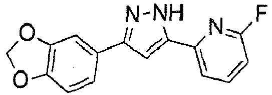 | 2-[3-(1,3-benzodioxol-5-yl)-1*H*-pyrazol-5-yl]-6-fluoropyridine | 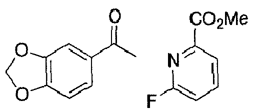 | A/55 |
| 12 | 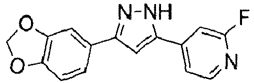 | 4-[3-(1,3-benzodioxol-5-yl)-1*H*-pyrazol-5-yl]-2-fluoropyridine | 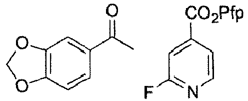 | D/76 |
| 13 | 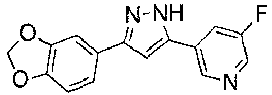 | 3-[3-(1,3-benzodioxol-5-yl)-1*H*-pyrazol-5-yl]-5-fluoropyridine | 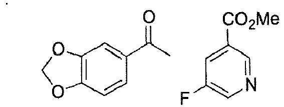 | A/65 |
| 14 | 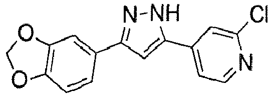 | 4-[3-(1,3-benzodioxol-5-yl)-1*H*-pyrazol-5-yl]-2-chloropyridine | 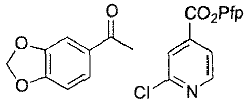 | D/60 |
| 15 | 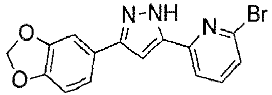 | 2-[3-(1,3-benzodioxol-5-yl)-1*H*-pyrazol-5-yl]-6-bromopyridine | 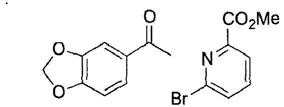 | A/67 |
| 16 | 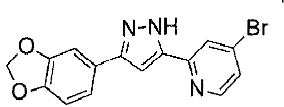 | 2-[3-(1,3-benzodioxol-5-yl)-1*H*-pyrazol-5-yl]-4-bromopyridine | 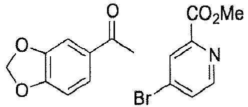 | B/80 |
| 17 | 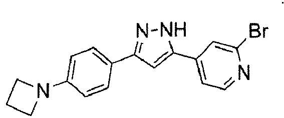 | 4-{3-[4-(azetidin-1-yl)phenyl]-1*H*-pyrazol-5-yl}-2-bromopyridine | 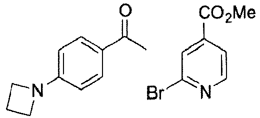 | A/40 |
| 18 | 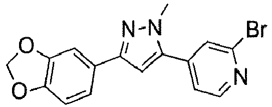 | 4-[3-(1,3-benzodioxol-5-yl)-1-methyl-1*H-*pyrazol-5-yl]-2-bromopyridine | 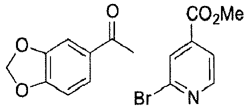 | A/34 |
| 19 | 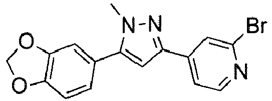 | 4-[5-(1,3-benzodioxol-5-yl)-1-methyl-1*H-*pyrazol-3-yl]-2-bromopyridine | 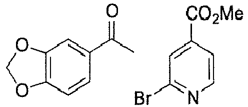 | A/23 |
| 20 | 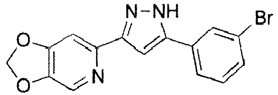 | 6-[3-(3-bromophenyl)-1*H*-pyrazol-5-yl]-1,3-dioxolo[4,5-c]pyridine | 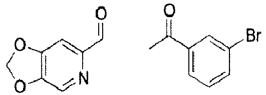 | F/32 |
| 21 | 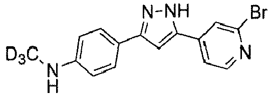 | 4-[5-(2-bromopyridin-4-yl)-1*H*-pyrazol-3-yl]-*N-*(²H₃)methylaniline | 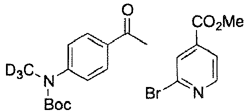 | B,C/60 |
| 22 | 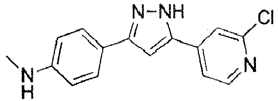 | 4-[5-(2-chloropyridin-4-yl)-1*H*-pyrazol-3-yl]-*N-*methylaniline | 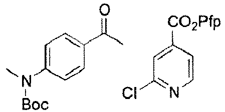 | D, C/32 |
| 23 | 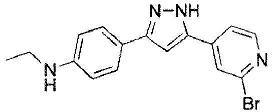 | 4-[5-(2-bromopyridin-4-yl)-1*H*-pyrazol-3-yl]-*N-*ethylaniline | 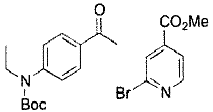 | B, C/62 |
| 24 | 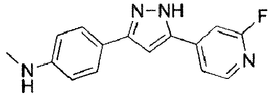 | 4-[5-(2-fluoropyridin-4-yl)-1*H*-pyrazol-3-yl]-*N-*methylaniline | 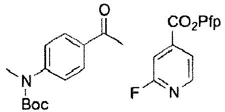 | D, C/32 |
| 25 | 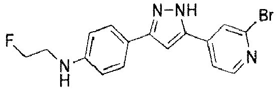 | 4-[5-(2-bromopyridin-4-yl)-1*H*-pyrazol-3-yl]-*N-*(2-fluoroethyl)aniline | 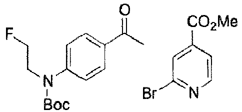 | A, C/46 |
| 26 | 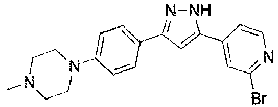 | 1 -{4-[5-(2-bromopyridin-4-yl)-1*H*-pyrazol-3-yl]phenyl}-4-methylpiperazine | 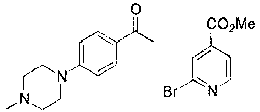 | B/63 |
| 27 | 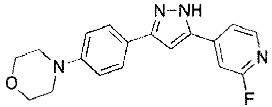 | 4-{4-[5-(2-fluoropyridin-4-yl)-1*H*-pyrazol-3-yl]phenyl}morpholine | 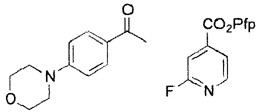 | D/79 |
| 28 | 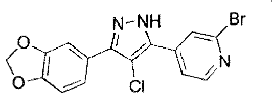 | 4-[3-(1,3-benzodioxol-5-yl)-4-chloro-1*H-*pyrazol-5-yl]-2-bromopyridine | 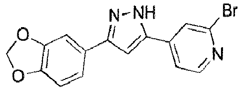 | K/47 |
| 29 | 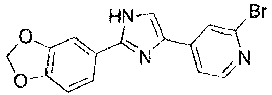 | 4-[2-(1,3-benzodioxol-5-yl)-1*H*-imidazol-4-yl]-2-bromopyridine | 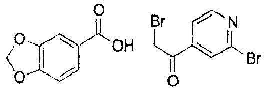 | H/26 |
| 30 | 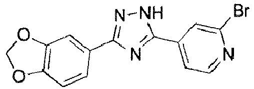 | 4-[3-(1,3-benzodioxol-5-yl)-1*H*-1,2,4-triazol-5-yl]-2-bromopyridine | 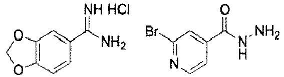 | I/49 |
| 31 | 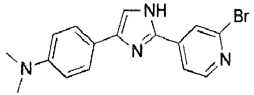 | 4-[2-(2-bromopyridin-4-yl)-1*H*-imidazol-4-yl]-*N,N*-dimethylaniline | 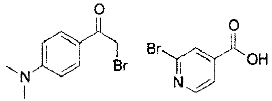 | H/25 |
| 32 | 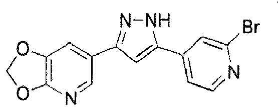 | 6-[5-(2-bromopyridin-4-yl)-1*H*-pyrazol-3-yl]-1,3-dioxolo[4,5-b]pyridine | 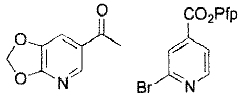 | D/54 |
| 33 | 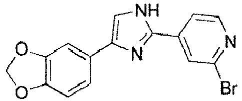 | 4-[4-(1,3-benzodioxol-5-yl)-1*H*-imidazol-2-yl]-2-bromopyridine | 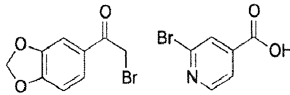 | H/36 |
| 34 | 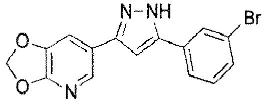 | 6-[5-(3-bromophenyl)-1*H*-pyrazol-3-yl]-1,3-dioxolo[4,5-b]pyridine | 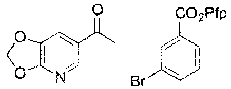 | D/33 |
| 35 | 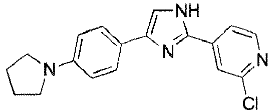 | 2-chloro-4-{4-[4-(pyrrolidin-1-yl)phenyl]-1*H*-imidazol-2-yl}pyridine | 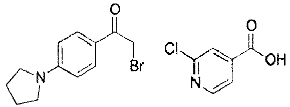 | H/20 |
| 36 | 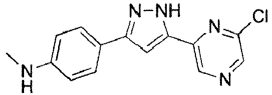 | 4-[5-(6-chloropyrazin-2-yl)-1*H*-pyrazol-3-yl]-*N-*methylaniline | 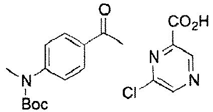 | G, C/21 |
| 37 | 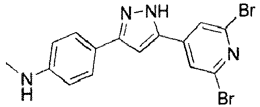 | 4-[5-(2,6-dibromopyridin-4-yl)-1*H*-pyrazol-3-yl]*-N-*methylaniline | 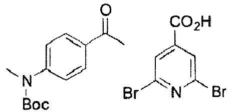 | G, C/49 |
| 38 | 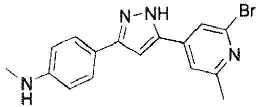 | 4-[5-(2-bromo-6-methylpyridin-4-yl)-1*H-*pyrazol-3-yl]-*N-*methylaniline | 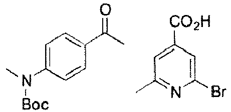 | G, C/28 |
| 39 | 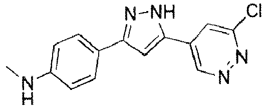 | 4-[5-(6-chloropyridazin-4-yl)-1*H*-pyrazol-3-yl]-*N*-methylaniline | 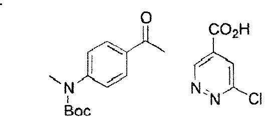 | G, C/44 |
| 40 | 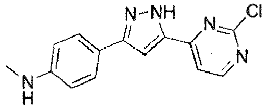 | 4-[5-(2-chloropyrimidin-4-yl)-1*H*-pyrazol-3-yl]-*N*-methylaniline | 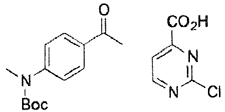 | G, C/10 |
| 41 | 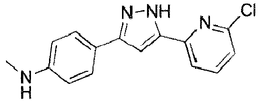 | 4-[5-(6-chloropyridin-2-yl)-1*H*-pyrazol-3-yl]-*N-*methylaniline | 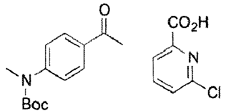 | G, C/30 |
| 42 | 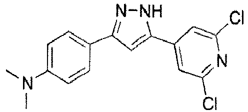 | 4-[5-(2,6-dichloropyridin-4-yl)-1*H*-pyrazol-3-yl]-*N*,*N-*dimethylaniline | 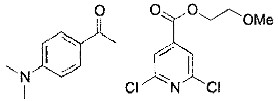 | A/34 |
| 43 | 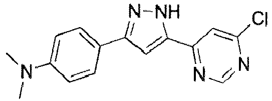 | 4-[5-(6-chloropyrimidin-4-yl)-1*H*-pyrazol-3-yl]-*N*,*N*-dimethylaniline | 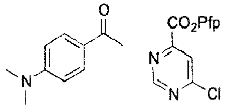 | D/9 |
| 44 | 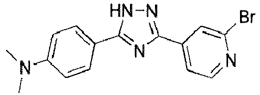 | 4-[3-(2-bromopyridin-4-yl)-1*H*-1,2,4-triazol-5-yl]-*N*,*N*-dimethylaniline | 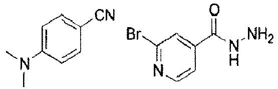 | J/8 |
| 45 | 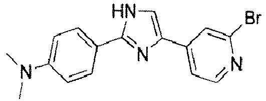 | 4-[4-(2-bromopyridin-4-yl)-1*H*-imidazol-2-yl]-*N*,*N*-dimethylaniline | 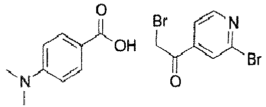 | H/14 |
| 46 | 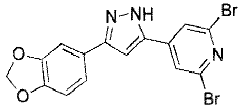 | 4-[3-(1,3-benzodioxol-5-yl)-1*H*-pyrazol-5-yl]-2,6-dibromopyridine | 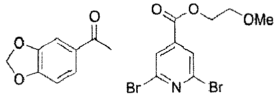 | A/14 |
| 47 | 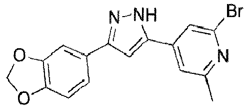 | 4-[3-(1,3-benzodioxol-5-yl)-1*H*-pyrazol-5-yl]-2-bromo-6-methylpyridine | 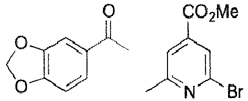 | A/50 |
| 48 | 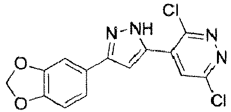 | 4-[5-(1,3-benzodioxol-5-yl)-1*H*-pyrazol-3-yl]-3,6-dichloropyridazine | 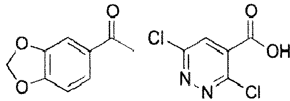 | G/36 |
| 49 | 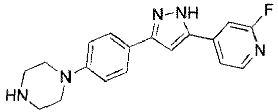 | 1-{4-[5-(2-fluoropyridin-4-yl)-1*H*-pyrazol-3-yl]phenyl}piperazine | 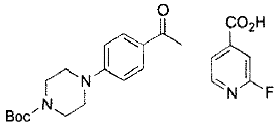 | G, C/10 |
| 50 | 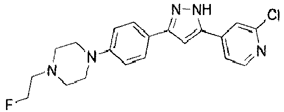 | 1-{4-[5-(2-chloropyrid in-4-yl)-1*H*-pyrazol-3-yl]phenyl}-4-(2-fluoroethyl)piperazine | 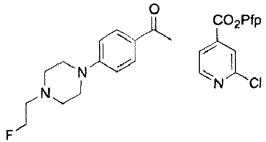 | D/29 |
| 51 | 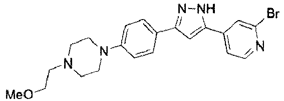 | 1-{4-[5-(2-bromopyridin-4-yl)-1*H*-pyrazol-3-yl]phenyl}-4-(2-methoxyethyl)piperazin e | 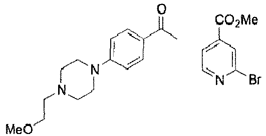 | B/70 |
| 52 | 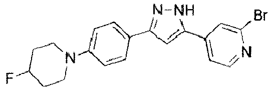 | 2-bromo-4-{3-[4-(4-fluoropiperidin-1-yl)phenyl]-1*H*-pyrazol-5-yl}pyridine | 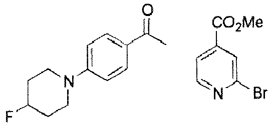 | B/61 |
| 53 | 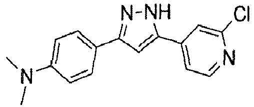 | 4-[5-(2-chloropyridin-4-yl)-1*H*-pyrazol-3-yl]-*N*,*N*-dimethylaniline | 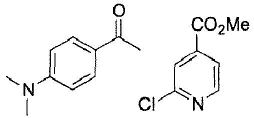 | B/66 |
| 54 | 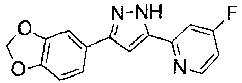 | 2-[3-(1,3-benzodioxol-5-yl)-1*H*-pyrazol-5-yl]-4-fluoropyridine | 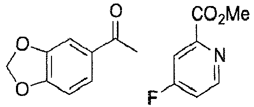 | B/18 |
| 55 | 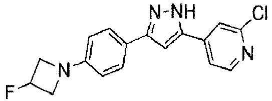 | 2-chloro-4-{3-[4-(3-fluoroazetidin-1-yl)phenyl]-1*H*-pyrazol-5-yl}pyridine | 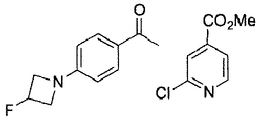 | B/85 |
| 56 | 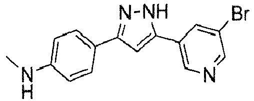 | 4-[5-(5-bromopyridin-3-yl)-1*H*-pyrazol-3-yl]-*N-*methylaniline | 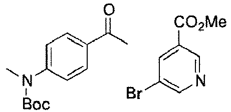 | B,C/31 |
| 57 | 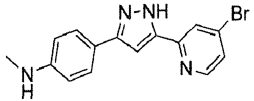 | 4-[5-(4-bromopyridin-2-yl)-1*H*-pyrazol-3-yl]-*N-*methylaniline | 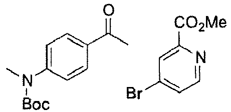 | B,C/31 |
| 58 | 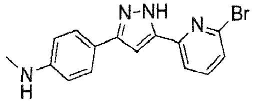 | 4-[5-(6-bromopyridin-2-yl)-1*H*-pyrazol-3-yl]-*N-*methylaniline | 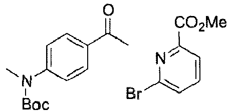 | B,C/49 |

**Table 2:**

| **Comp. no.** | **HPLC Retention Time (min)** | **Mass Spec [M+H]⁺** | **¹H-NMR Data (ppm) δ (solvent, T)** |
|---|---|---|---|
| 1 | 4.8^{a} | 343.1 (100%), 345.2 (97%) | 8.42 (d, *J* = 5.2 Hz, 1H), 8.05 (d, *J* = 1.4 Hz, 1H), 7.93 (d, *J* = 8.7 Hz, 2H), 7.87 (dd, *J* = 5.2, 1.4 Hz, 1H), 7.66 (bd, *J* = 7.8 Hz, 2H), 7.51 (s, 1H), 3.11 (s, 6H) |
| | | | (DMSO-d6 + 1% DCI, 298K) |
| 2 | 4.0^{a} | 329.1 (98%), 331.1 (100%) | 8.41 (d, *J* = 5.2 Hz, 1H), 8.04 (d, *J* = 1.1 Hz, 1H), 7.93 (d, *J* = 8.6 Hz, 2H), 7.86 (dd, *J* = 5.2, 1.4 Hz, 1H), 7.57 (d, *J* = 8.6 Hz, 2H), 7.48 (s, 1H), 2.92 (s, 3H) |
| | | | (DMSO-d6 + 1% DCI, 313K) |
| 3 | 3.9^{a} | 283.2 (100%) | 8.28 (d, *J* = 5.2 Hz, 1H), 7.93 (bd, *J* = 8.7 Hz, 2H), 7.78 (dt, *J* = 5.2, 1.6 Hz, 1H), 7.67 (bd, *J* = 8.7 Hz, 2H), 7.55 (s, 1H), 7.45 (s, 1H), 3.12 (s, 6H) |
| | | | (DMSO-d6 + 1% DCI, 313K) |
| 4 | 3.6^{a} | 344.1 (98%), 346.1 (100%) | 8.71 (dd, *J* = 6.2, 1.1 Hz, 1H), 8.55 (s, 1H), 8.35 (m, 2H), 8.27 (d, *J* = 9.6 Hz, 1H), 7.41 (d, *J* = 1.1 Hz, 1H), 7.26 (d, *J* = 9.6 Hz, 1H), 3.38 (s, 6H) |
| | | | (TFA-d1, 313K) |
| 5 | 9.2^{a} | 344.1 (100%), 346.1 (98%) | 9.07 (s, 2H), 7.82 (s, 1H), 7.60 (d, *J* = 8.0 Hz, 2H), 7.29 (t, *J* = 8.0 Hz, 1H), 7.26 (s, 1H), 3.37 (s, 6H) |
| | | | (TFA-d1, 298K) |
| 6 | 6.2^{a} | 345.1 (100%), 347.3 (98%) | 8.70 (s, 2H), 8.46 (d, *J* = 6.4 Hz, 1H), 8.30 (s, 1H), 8.10 (dd, *J* = 6.4, 1.6 Hz, 1H), 7.24 (s, 1H), 3.19 (s, 6H) |
| | | | (TFA-d1, 298K) |
| 7 | 3.3^{a} | 315.1 (98%), 317.1 (100%) | 13.22 (bs, 1H), 8.39 (d, *J* = 5.2 Hz, 1H), 8.01 (d, *J* = 0.8 Hz, 1H), 7.83 (dd, *J* = 5.2, 1.4 Hz, 1H), 7.47 (ddd, *J* = 7.5, 1.7, 0.9 Hz, 2H), 7.19 (s, 1H), 6.64 (ddd, *J* = 7.5, 1.7, 0.9 Hz, 2H), 5.49 (bs, 2H) |
| | | | (DMSO-d6, 298K) |
| 8 | 5.2^{a} | 285.2 (100%) | 8.69 (s, 2H), 8.31 (dd, *J* = 6.4, 2.0 Hz, 1H), 7.94 (dd, *J* = 6.4, 1.3 Hz, 1H), 7.76 (dd, *J* = 3.2, 1.3 Hz, 1H), 7.22 (s, 1H), 3.17 (s, 6H) |
| | | | (TFA-d1, 298K, two forms in ratio 10/1, the main form) |
| 9 | 10.4^{a} | 344.1 (100%), 346.2 (98%) | 8.41 (d, *J* = 5.1 Hz, 1H), 8.01 (d, *J* = 1.2 Hz, 1H), 7.84 (dd, *J* = 5.1, 1.5 Hz, 1H), 7.38 (d, *J* = 1.7 Hz, 1H), 7.34 (s, 1H), 7.33 (dd, *J* = 8.1, 1.7 Hz, 1H), 7.00 (d, *J* = 8.1 Hz, 1H), 6.06 (s, 2H) |
| | | | (DMSO-d6 + 1% DCI, 313K) |
| 10 | 9.8^{a} | 344.1 (100%), 346.2 (98%) | 9.05 (d, *J* = 1.2 Hz, 1H), 8.67 (d, *J* = 2.0 Hz, 1H), 8.46 (bs, 1H), 7.37 (d, *J* = 2.0 Hz, 1H), 7.32 (dd, *J* = 8.1, 1.5 Hz, 1H), 7.27 (s, 1H), 6.99 (d, *J* = 8.1 Hz, 1H), 6.06 (s, 2H) |
| | | | (DMSO-d6 + 1% DCI, 313K) |
| 11 | 8.7^{a} | 284.2 | 8.04 (q, *J* = 8.0 Hz, 1H), 7.89 (dd, *J* = 7.5, 2.4 Hz, 1H), 7.44 (d, *J* = 1.7 Hz, 1H), 7.38 (dd, *J* = 8.1, 1.7 Hz, 1H), 7.22 (s, 1H), 7.09 (dd, *J* = 8.0, 2.4 Hz, 1H), 6.98 (d, *J* = 8.1 Hz, 1H), 6.05 (s, 2H) |
| | | | (DMSO-d6 + 1% DCI, 298K) |
| 12 | 8.4^{a} | 284.2 (100%) | 8.26 (d, *J* = 5.2 Hz, 1H), 7.76 (dt, *J* = 5.2, 1.7 Hz, 1H), 7.53 (s, 1H), 7.39 (d, *J* = 1.7 Hz, 1H), 7.37 (s, 1H), 7.33 (dd, *J* = 8.1, 1.8 Hz, 1H), 7.01 (d, *J* = 8.1 Hz, 1H), 6.06 (s, 2H) |
| | | | (DMSO-d6 + 1% DCI) |
| 13 | 7.4^{a} | 284.2 | 8.98 (t, *J* = 1.7 Hz, 1H), 8.62 (d, *J* = 2.7 Hz, 1H), 8.21 (ddd, *J* = 8.1, 2.7, 1.7 Hz, 1H), 7.37 (d, *J* = 1.7 Hz, 1H), 7.32 (dd, *J* = 8.1, 1.8 Hz, 1H), 7.32 (s, 1H), 7.01 (d, *J* = 8.1 Hz, 1H), 6.07 (s, 2H) |
| | | | (DMSO-d6 + 1% DCI, 298K) |
| 14 | 9.8^{a} | 300.1 (100%) | 8.43 (d, *J* = 5.2 Hz, 1H), 7.87 (s, 1H), 7.81 (dd, *J* = 5.2, 1.4 Hz, 1H), 7.39 (d, *J* = 1.6 Hz, 1H), 7.34 (s, 1H), 7.34 (dd, *J* = 8.2, 1.6 Hz, 1H), 7.00 (d, *J* = 8.1 Hz, 1H), 6.06 (s, 2H) |
| | | | (DMSO-d6 + 1% DCI, 313K) |
| 15 | 11.2^{a} | 344.2 (100%), 346.2 (97%) | 7.97 (d, *J* = 7.8 Hz, 1H), 7.80 (t, *J* = 7.8 Hz, 1H), 7.55 (d, *J* = 7.8 Hz, 1H), 7.45 (d, *J* = 1.6 Hz, 1H), 7.39 (dd, *J* = 8.1, 1.6 Hz, 1H), 7.20 (s, 1H), 6.97 (d, *J* = 8.1 Hz, 1H), 6.04 (s, 2H) |
| | | | (DMSO-d6 + 1% DCI, 298K) |
| 16 | 7.3^{a} | 344.0 (100%), 346.0 (97%) | 9.47 (d, *J* = 1.7 Hz, 1H), 9.44 (d, *J* = 6.4 Hz, 1H), 9.04 (dd, *J* = 6.4, 1.7 Hz, 1H), 8.21 (s, 1H), 8.12 (dd, *J* = 8.1, 1.7 Hz, 1H), 8.04 (d, *J* = 1.7 Hz, 1H), 7.80 (d, *J* = 8.1 Hz, 1H), 6.86 (s, 2H) |
| | | | (TFA-d1, 298K) |
| 17 | 7.5^{a} | 355.2 (100%), 357.2 (98%) | 8.40 (d, *J* = 5.2 Hz, 1H), 8.02 (d, *J* = 1.4 Hz, 1H), 7.84 (dd, *J* = 5.2, 1.4 Hz, 1H), 7.74 (d, *J* = 8.6 Hz, 2H), 7.31 (s, 1H), 7.11 (bd, *J* = 8.4 Hz, 2H), 3.76 (t, *J* = 7.0 Hz, 2H), 3.31 (t, *J* = 7.0 Hz, 2H), 2.10 (qi, *J* = 7.0 Hz, 2H) |
| | | | (DMSO-d6 + 1% DCI, 313K) |
| 18 | 12.6^{a} | 358.2 (100%), 360.2 (98%) | 8.51 (d, *J* = 5.1 Hz, 1H), 7.89 (d, *J* = 0.7 Hz, 1H), 7.69 (dd, *J* = 5.1, 1.3 Hz, 1H), 7.35 (s, 1H), 7.33 (d, *J* = 9.3 Hz, 1H), 7.12 (s, 1H), 6.96 (d, *J* = 7.9 Hz, 1H), 6.05 (s, 2H), 3.97 (s, 3H) |
| | | | (DMSO-d6, 298K) |
| 19 | 13.7^{a} | 358.1 (100%), 360.2 (98%) | 8.36 (d, *J* = 5.0 Hz, 1H), 7.90 (s, 1H), 7.64 (d, *J* = 4.7 Hz, 1H), 6.92 (s, 1H), 6.91 (d, *J* = 8.9 Hz, 2H), 6.61 (s, 1H), 6.06 (s, 2H), 3.92 (s, 3H) |
| | | | (CDCl₃, 298K) |
| 20 | 17.3^{b} | 344.2 (97%), 346.2 (100%) | 8.37 (s, 1H), 8.07 (t, *J* = 1.8 Hz, 1H), 8.03 (s, 1H), 7.89-7.82 (m, 2H), 7.61 (ddd, *J* = 8.0, 2.0, 0.9 Hz, 1H), 7.47 (t, *J* = 7.9 Hz, 1H), 6.52 (s, 2H) |
| | | | (DMSO-d6 + 1% DCI, 298K) |
| 21 | 17.7^{b} | 329.1 (98%), 331.1 (100%) | 8.46 (d, *J* = 6.4 Hz, 1H), 8.31 (s, 1H), 8.12 (d, *J* = 6.4 Hz, 1H), 7.70 (d, *J* = 8.6 Hz, 2H), 7.43 (d, *J* = 8.6 Hz, 2H), 7.22 (s, 1H) |
| | | | (TFA-d1, 298K) |
| 22 | 15.2^{b} | 285.2 (100%) | 8.46 (d, *J* = 5.2 Hz, 1H), 7.97 (d, *J* = 8.6 Hz, 2H), 7.94 (s, 1H), 7.85 (dd, *J* = 5.2, 1.4 Hz, 1H), 7.64 (d, *J* = 8.6 Hz, 2H), 7.56 (s, 1H), 2.92 (s, 3H) |
| | | 287.2 (34%) | |
| | | | (DMSO-d6 + 1% DCI, 298K) |
| 23 | 15.9^{b} | 343.2 (99%) | 8.43 (d, *J* = 5.2 Hz, 1H), 8.07 (d, *J* = 1.2 Hz, 1H), 7.98 (d, *J* = 8.6 Hz, 2H), 7.88 (dd, *J* = 5.2, 1.5 Hz, 1H), 7.69 (d, *J* = 8.6 Hz, 2H), 7.57 (s, 1H), 3.34 (q, *J* = 7.3 Hz, 2H), 1.27 (t *J* = 7.3 Hz, 3H) |
| | | 345.2 (100%) | |
| | | | (DMSO-d6 + 1% DCI, 298K) |
| 24 | 14.3^{b} | 269.2 (100%) | 8.30 (d, *J* = 5.3 Hz, 1H), 7.98 (d, *J* = 8.6 Hz, 2H), 7.80 (m, 1H), 7.66 (d, *J* = 8.6 Hz, 2H), 7.59 (s, 1H), 7.54 (s, 1H), 2.92 (s, 3H) |
| | | | (DMSO-d6 + 1% DCI, 298K) |
| 25 | 21.2^{b} | 361.3 (100%) | 8.42 (d, *J* = 5.2 Hz, 1H), 8.07 (d, *J* = 0.8 Hz, 1H), 7.92-7.84 (m, 3H), 7.49 (s, 1H), 7.41 (d, *J* = 8.6 Hz, 2H), 4.73 (dt, *J* = 47.3, 4.8 Hz, 2H), 3.61 (dt, *J* = 27.2, 4.8 Hz, 2H) |
| | | 363.2 (100%) | |
| | | | (DMSO-d6 + 1% DCI, 298K) |
| 26 | 15.9^{b} | 398.3 (99%) | 8.40 (d, *J* = 5.2 Hz, 1H), 8.05 (s, 1H), 7.87 (d, *J* = 5.2 Hz, 1H), 7.74 (d, *J* = *8.7* Hz, 2H), 7.39 (s, 1H), 7.11 (d, *J* = 8.7 Hz, 2H), 3.97-3.87 (m, 2H), 3.52-3.43 (m, 2H), 3.27-3.10 (m, 4H), 2.80 (s, 3H) |
| | | 400.4 (100%) | |
| | | | (DMSO-d6 + 1% DCI, 298K) |
| 27 | 19.5^{b} | 325.3 (100%) | 8.28 (d, *J* = 5.3 Hz, 1H), 7.88 (d, *J* = 8.8 Hz, 2H), 7.82-7.77 (m, 1H), 7.60-7.52 (m, 3H), 7.47 (s, 1H), 3.44 (m, 4H), 3.96 (m, 4H) |
| | | | (DMSO-d6 + 1% DCI, 298K) |
| 28 | 25.8^{b} | 378.0 (76%) | 8.49 (dd, *J* = 5.3, 0.6 Hz, 1H), 8.05 (dd, *J* = 1.5, 0.6 Hz, 1H), 7.93 (dd, *J* = 5.3, 1.4 Hz, 1H), 7.33-7.28 (m, 2H), 7.08 (d, *J* = 8.6 Hz, 1H), 6.10 (s, 2H) |
| | | 380.1 (100%) | |
| | | | (DMSO-d6 + 1% DCI, 298K) |
| 29 | 15.0^{b} | 344.1 (100%) | 8.52 (s, 1H), 8.51 (d, *J* = 5.3 Hz, 1H), 8.18 (s, 1H), 7.92 (d, *J* = 5.2, 1.3 Hz, 1H), 7.68-7.60 (m, 2H), 7.21 (d, *J* = 8.1 Hz, 1H), 6.19 (s, 2H) |
| | | 346.1 (98%) | |
| | | | (DMSO-d6 + 1% DCI, 298K) |
| 30 | 22.6^{b} | 345.2 (100%) | 8.50 (d, *J* = 5.1 Hz, 1H), 8.14 (s, 1H), 8.01 (d, *J* = 5.1 Hz, 1H), 7.66 (dd, *J* = 8.1, 1.6 Hz, 1H), 7.61 (d, *J* = 1.6 Hz, 1H), 7.08 (d, *J* = 8.1 Hz, 1H), 6.12 (s, 2H) |
| | | 347.2 (93%) | |
| | | | (DMSO-d6 + 1% DCI, 298K) |
| 31 | 13.3^{b} | 343.2 (100%) | 8.59 (dd, *J* = 5.2, 0.6 Hz, 1H), 8.28 (dd, *J* = 1.6, 0.6 Hz, 1H), 8.07 (s, 1H), 8.03 (dd, *J* = 5.3, 1.6 Hz, 1H), 7.75 (d, *J* = 8.9 Hz, 2H), 6.94 (d, *J* = 8.9 Hz, 2H), 3.00 (s, 6H) |
| | | 345.1 (98%) | |
| | | | (DMSO-d6 + 1% TFA-d1, 298K) |
| 32 | 20.4^{b} | 345.1 (99%) | 8.43 (d, *J* = 5.3 Hz, 1H), 8.12 (d, *J* = 1.9 Hz, 1H), 8.03 (s, 1H), 7.84 (dd, *J* = 5.2, 1.4 Hz, 1H), 7.66 (d, *J* = 1.8 Hz, 1H), 7.49 (s, 1H), 6.20 (s, 2H) |
| | | 347.1 (100%) | |
| | | | (DMSO-d6 + 1% DCI, 298K) |
| 33 | 16.2^{b} | 344.2 (100%) | 8.60 (dd, *J* = 5.2, 0.6 Hz, 1H), 8.27 (dd, *J* = 1.5, 0.6 Hz, 1H), 8.12 (s, 1H), 8.02 (dd, *J* = 5.2, 1.5 Hz, 1H), 7.46 (d, *J* = 1.8 Hz, 1H), 7.41 (dd, *J* = 8.1, 1.8 Hz, 1H), 7.06 (d, *J* = 8*.*1 Hz, 1H), 6.09 (s, 2H) |
| | | 346.2 (96%) | |
| | | | (DMSO-d6 + 1% TFA-d1, 298K) |
| 34 | 24.1^{b} | 344.1 (96%) | 8.12 (d, *J* = 1.8 Hz, 1H), 8.03 (t, *J* = 1.8 Hz, 1H), 7.83 (d, *J* = 7.7 Hz, 1H), 7.66 (d, *J* = 1.8 Hz, 1H), 7.53 (m, 1H), 7.42 (t, *J* = 7.9 Hz, 1H), 7.30 (s, 1H), 6.19 (s, 2H) |
| | | 346.1 (100%) | |
| | | | (DMSO-d6 + 1% DCI, 298K) |
| 35 | 17.8^{b} | 325.3 (100%) | 8.71 (dd, *J* = 5.3, 0.6 Hz, 1H), 8.21 (dd, *J* = 1.6, 0.6 Hz, 1H), 8.18 (s, 1H), 8.04 (dd, *J* = 5.3, 1.6 Hz, 1H), 7.71 (d, *J* = 8.9 Hz, 2H), 6.69 (d, *J* = 8.9 Hz, 2H), 3.30 (m, 4H), 1,98 (m, 4H) |
| | | 327.4 (35%) | |
| | | | (DMSO-d6 + 1% TFA-d1, 298K) |
| 36 | 15.0^{b} | 286.2 (100%) | 9.20 (s, 1H), 8.72 (s, 1H), 8.04 (d, *J* = 8.7 Hz, 2H), 7.64 (d, *J* = 8.7 Hz, 2H), 7.49 (s, 1H), 2.92 (s, 3H) |
| | | 288.2 (33%) | |
| | | | (DMSO-d6 + 1% DCI, 298K) |
| 37 | 19.3^{b} | 407.1 (52%) | 8.13 (s, 2H), 7.96 (d, *J* = 8.6 Hz, 2H), 7.66 (d, *J* = 8.6 Hz, 2H), 7.65 (s, 1H), 2.91 (s, 3H) |
| | | 409.1 (100%) | |
| | | 411.1 (50%) | (DMSO-d6 + 1% DCI, 298K) |
| 38 | 16.3^{b} | 343.2 (100%) | 7.94 (d, *J* = 8.7 Hz, 2H), 7.86 (s, 1H), 7.76 (s, 1H), 7.58 (d, *J* = 8.7 Hz, 2H), 7.50 (s, 1H), 2.91 (s, 3H), 2.49 (s, 3H) |
| | | 345.1 (98%) | |
| | | | (DMSO-d6 + 1% DCI, 298K) |
| 39 | 13.4^{b} | 286.2 (100%) | 9.71 (d, *J* = 1.8 Hz, 1H), 8.26 (d, *J* = 1.8 Hz, 1H), 7.94 (d, *J* = 8.6 Hz, 2H), 7.67 (s, 1H), 7.61 (d, *J* = 8.6 Hz, 2H), 2.92 (s, 3H) |
| | | 288.2 (34%) | |
| | | | (DMSO-d6 + 1% DCI, 298K) |
| 40 | 14.2^{b} | 286.2 (100%) | 8.80 (d, *J* = 5.2 Hz, 1H), 8.09-8.01 (m, 3H), 7.64 (d, *J* = 8.6 Hz, 2H), 7.54 (s, 1H), 2.92 (s, 3H) |
| | | 288.2 (30%) | |
| | | | (DMSO-d6 + 1% DCI, 298K) |
| 41 | 15.2^{b} | 285.2 (100%) | 8.02 (d, *J* = 8.7 Hz, 2H), 7.99-7.92 (m, 2H), 7.59 (d, *J* = 8.7 Hz, 2H), 7.46 (dd, *J* = 6.7, 2.0 Hz, 1H), 7.37 (s, 1H), 2.92 (s, 3H) |
| | | 287.2 (34%) | |
| | | | (DMSO-d6 + 1% DCI, 298K) |
| 42 | 19.6^{b} | 333.1 (100%) | 8.01-7.96 (m, 4H), 7.89 (d, *J* = 8.8 Hz, 2H), 7.67 (s, 1H), 3.14 (s, 6H) |
| | | 334.1 (61%) | |
| | | 335.1 (61%) | (DMSO-d6 + 1% DCI, 298K) |
| 43 | 15.4^{b} | 300.2 (100%) | 9.02 (s, 1H), 8.05 (s, 1H), 7.65 (d, *J* = 8.6 Hz, 2H), 7.24 (s, 1H), 6.78 (d, *J* = 8.6 Hz, 2H), 2.94 (s, 6H) |
| | | 302.2 (33%) | |
| | | | (DMSO-d6 + 2% acetic acid-d4, 298K) |
| 44 | 19.2^{b} | 344.2 (100%) | 8.53 (d, *J* = 5.1 Hz, 1H), 8.27 (d, *J* = 8.8 Hz, 2H), 8.24 (s, 1H), 8.08 (dd, *J* = 5.1, 1.3 Hz, 1H), 7.76 (d, *J* = 8.6 Hz, 2H), 3.13 (s, 6H) |
| | | 346.3 (100%) | |
| | | | (DMSO-d6 + 1% DCI, 298K) |
| 45 | 16.9^{b} | 343.2 (100%) | 8.50 (d, *J* = 5.3 Hz, 1H), 8.48 (s, 1H), 8.20 (d, *J* = 1.0 Hz, 1H), 7.97-7.91 (m, 3H), 6.89 (d, *J* = 9.1 Hz, 2H), 3.04 (s, 6H) |
| | | 345.2 (98%) | |
| | | | (DMSO-d6 + 1% TFA-d1, 298K) |
| 46 | 26.5^{b} | 422.1 (52%) | 8.08 (s, 2H), 7.49 (s, 1H), 7.38 (d, *J* = 1.6 Hz, 1H), 7.33 (dd, *J* = 8.1, 1.6 Hz, 1H), 7.03 (d, *J* = 8.1 Hz, 1H), 6.07 (s, 2H) |
| | | 424.1 (100%) | |
| | | 426.1 (49%) | (DMSO-d6 + 1% DCI, 298K) |
| 47 | 24.2^{b} | 358.1 (100%) | 7.84 (s, 1H), 7.75 (s, 1H), 7.39 (d, *J* = 1.7 Hz, 1H), 7.37 (s, 1H), 7.33 (dd, *J* = 8.1, 1.7 Hz, 1H), 7.01 (d, *J* = 8.1 Hz, 1H), 6.06 (s, 2H), 2.49 (s, 3H) |
| | | 360.1 (98%) | |
| | | | (DMSO-d6 + 1% DCI, 298K) |
| 48 | 22.5^{b} | 335.1 (100%) | 8.31 (s, 1H), 7.48-7.45 (m, 2H), 7.38 (dd, *J* = 8.1, 1.8 Hz, 1H), 7.03 (d, *J* = 8.1 Hz, 1H), 6.08 (s, 2H) |
| | | 337.2 (66%) | |
| | | | (DMSO-d6 + 1% DCI, 298K) |
| 49 | 14.9^{b} | 324.3 (100%) | 9.02-8.84 (m, 1H), 8.28 (d, *J* = 5.2 Hz, 1H), 7.78 (d, *J* = 5.1 Hz, 1H), 7.71 (d, *J* = 8.7 Hz, 2H), 7.54 (s, 1H), 7.34 (s, 1H), 7.11 (d, *J* = 8.7 Hz, 2H), 3.48-3.40 (m, 4H), 3.30-3.21 (m, 4H) |
| | | | (DMSO-d6 + 1% TFA-d1, 298K) |
| 50 | 16.3^{b} | 386.3 (100%) | 8.43 (d, *J* = 5.2 Hz, 1H), 7.91 (s, 1H), 7.84 (dd, *J* = 5.2, 1.3 Hz, 1H), 7.74 (d, *J* = 8.8 Hz, 2H), 7.38 (s, 1H), 7.11 (d, *J* = 8.8 Hz, 2H), 4.96 (dt, *J* = 47.2, 4.3 Hz, 2H), 4.00-3.88 (m, 2H), 3.67-3.50 (m, 4H), 3.34-3.20 (m, 4H) |
| | | 388.3 (37%) | |
| | | | (DMSO-d6 + 1% DCI, 298K) |
| 51 | 17.1^{b} | 442.4 (93%) | 8.41 (d, *J* = 5.2 Hz, 1H), 8.04 (s, 1H), 7.87 (dd, *J* = 5.2, 1.2 Hz, 1H), 7.73 (d, *J* = 8.8 Hz, 2H), 7.38 (s, 1H), 7.11 (d, *J* = 8.8 Hz, 2H), 3.96-3.87 (m, 2H), 3.78 (t, *J* = 4.6 Hz, 2H), 3.61-3.52 (m, 2H), 3.36 (t, *J* = 4.6 Hz, 2H), 3.31 (s, 3H), 3.29-3.15 (m, 4H) |
| | | 444.2 (100%) | |
| | | | (DMSO-d6 + 1% DCI, 298K) |
| 52 | 19.1^{b} | 401.2 (100%) | 8.43 (d, *J* = 5.2 Hz, 1H), 8.07 (s, 1H), 8.00 (s, 4H), 7.88 (dd, *J* = 5.3, 1.3 Hz, 1H), 7.60 (s, 1H), 5.17-4.96 (m, 1H), 3.79-3.53 (m, 4H), 2.57-2.36 (m, 2H), 2.34-2.17 (m, 2H) |
| | | 403.2 (98%) | |
| | | | (DMSO-d6 + 1% DCI, 298K) |
| 53 | 4.7^{a} | 299.3 (100%) | 8.46 (dd, *J* = 5.2, 0.2 Hz, 1H), 8.00 (ddd, *J* = 8.8, 2.2, 2.2 Hz, 2H), 7.94 (d, *J* = 0.9 Hz, 1H), 7.89-7.79 (m, 3H), 7.58 (s, 1H), 3.14 (s, 6H) |
| | | | (DMSO-d6 + 1% DCI, 298K) |
| 54 | 17.0^{b} | 284.2 (100%) | 8.79 (t, *J* = 6.5 Hz, 1H), 8.19 (dd, *J* = 9.3, 2.5 Hz, 1H), 7.67 (ddd, *J* = 7.8, 6.5, 2.5, Hz, 1H), 7.60 (s, 1H), 7.42 (d, *J* = 1.8 Hz, 1H), 7.35 (dd, *J* = 8.1, 1.8 Hz, 1H), 7.04 (d, *J* = 8.1 Hz, 1H), 6.08 (s, 2H) |
| | | | (DMSO-d6 + 1% DCI, 298K) |
| 55 | 12.2^{a} | 329.2 (100%) | 8.42 (d, *J* = 5.2 Hz, 1H), 7.88 (s, 1H), 7.82 (dd, *J* = 5.2, 1.4, Hz, 1H), 7.64 (ddd, *J* = 8.6, 2.3, 2.3 Hz, 2H), 7.30 (bs, 1H), 6.58 (ddd, *J* = 8.6, 2.3, 2.3 Hz, 2H), 5.58 and 5.43 (dtt, *J* = 57.6, 5.8, 3.1 Hz, 2×0.5 H), 4.20 (dddd, *J* = 20.7, 9.4, 5.7, 1.0 Hz, 2H), 3.93 (dddd, *J* = 24.3, 9.4, 3.1, 1.2 Hz, 2H) |
| | | 331.1 (33%) | |
| | | | (DMSO-d6, 298K) |
| 56 | 15.3^{b} | 329.2 (100%) | 9.08 (d, *J* = 1.8 Hz, 1H), 8.71 (d, *J* = 2.2 Hz, 1H), 8.53 (t, *J* = 2.0 Hz, 1H), 7.91 (bd, *J* = 8.6 Hz, 2H), 7.50 (bd, *J* = 8.6 Hz, 2H), 7.44 (s, 1H), 2.90 (s, 3H) |
| | | 331.1 (98%) | |
| | | | (DMSO-d6 + 1% DCI, 298K) |
| 57 | 14.6^{b} | 329.2 (100%) | 8.48 (d, *J* = 5.3 Hz, 1H), 8.15 (d, *J* = 1.8 Hz, 1H), 7.57 (d, *J* = 8.6 Hz, 2H), 7.56 (m, 1H), 7.08 (s, 1H), 6.60 (bd, *J* = 8.6 Hz, 2H), 2.71 (s, 3H) |
| | | 331.1 (98%) | |
| | | | (DMSO-d6, 298K) |
| 58 | 16.4^{b} | 329.2 (100%) | 8.02-7.98 (m, 3H), 7.83 (t, *J* = 7.8 Hz, 1H), 7.60-7.57 (m, 3H), 7.35 (s, 1H), 2.92 (s, 3H) |
| | | 331.1 (98%) | |
| | | | (DMSO-d6 + 1% DCI, 298K) |

| | | | |
|---|---|---|---|
| ^{a} gradient 50% CH₃CN /50% H₂O → 100% CH₃CN in 30 min ^{b} gradient 5% CH₃CN /100% H₂O → 100% CH₃CN in 30 min | | | |

### Example 2: Binding studies with aggregated alpha-synuclein, tau, and Abeta

To analyze the binding affinities and target selectivity of compounds, two types of *in vitro* fibril binding assays were used: a saturation assay and a competition assay.

### 1) Preparation of fibrils

Alpha-synuclein and tau46 were purified from *E. coli* according to established protocols (Nuscher B, et al., J. Biol. Chem., 2004; 279(21) :21966-75), Aβ₁₋₄₂ was obtained from rPeptide, Watkinsville, GA, USA. Fibrils were prepared by aggregation of recombinant protein via constant agitation. In detail, 70 µM of alpha-synuclein mixed with 100 mM NaCl in 50 mM Tris, pH 7.0 + 0.02% NaN₃ were incubated at 1,400 rpm, 37°C for 96 h. 10 µM of tau46 were incubated with 0.03 mg/ml heparin in 50 mM Tris, pH = 7.0 at 1,000 rpm, 37°C for 72 h. Aβ₁₋₄₂ was solved in 20 mM NaPᵢ, pH = 8.0 + 0.2 mM EDTA + 0.02% NaN₃ (Deeg AA, et al., Biochim. Biophys. Acta, 2015;1850(9): 1884-90; Goedert M, et al. Nature, 1996; 383(6600): 550-3).

### 2a) Saturation binding assay

Sonicated αSYN (0.04 µM) or tau46 (0.4 µM) or Aβ₁₋₄₂ (6 µM) fibrils diluted in PBS were incubated with decreasing concentrations of [³H]-labeled compound (48 nM or 24 nM - 23 pM) in 50 mM Tris-base, 10% ethanol, 0.05% Tween20, pH 7.4. To determine non-specific binding, the respective unlabeled compound (400 nM) was added to a duplicate set of binding reactions.

Assay plates were incubated under agitation at 37°C for 2 h, covered by plastic foil (Resealable tape, PerkinElmer, Waltham, MA, USA). Prior to harvesting, a filter (Printed filtermat B, PerkinElmer, Waltham, MA, USA) was incubated with 5 mg/mL polyethylenimine (PEI, Poly(ethyleneimine) solution, Sigma Aldrich Chemie GmbH, Taufkirchen, Germany) at 4°C for 30 min. After incubation, bound and free ligands were separated by vacuum filtration using a harvester (Filtermate harvester, PerkinElmer, Waltham, MA, USA). The filter was washed three times with buffer cooled to 4°C and subsequently dried in the microwave oven for 2 min at medium power. Melt-on scintillator sheets (MeltiLex^{™} B/HS, PerkinElmer, Waltham, MA, USA) were molten into the filter using a heating plate set to 120°C. After solidifying at room temperature, the filter was sealed in a plastic bag (Sample bag for MicroBeta^{®}, PerkinElmer, Waltham, MA, USA). Accumulation of tritium was counted in a liquid scintillation counter (Wallac MicroBeta^{®} TriLux, 1450 LSC & Luminescence Counter, PerkinElmer, Waltham, MA, USA) immediately. Radioactivity was plotted against the increasing tritium-labeled compound or cold compound concentrations. Data points were fitted using nonlinear regression analysis in GraphPad Prism (GraphPad Software, Inc., Version 7.03, La Jolla, CA, USA).

### 2b) Modified saturation binding assay

For saturation binding assays, a fixed concentration of sonicated human recombinant αSYN (15 nM/well) or tau46 (250 nM/well) or Aβ₁₋₄₂ (1 µM/well) fibrils diluted in phosphate buffered saline (PBS) were incubated in low binding plates (96 well micro test plate, Ratiolab GmbH, Dreieich, Germany) with [³H]-compound 1 or [³H]-compound 2 at increasing concentrations (from 0.05 nM to 12 nM/24 nM) in 30 mM Tris HCl, 10 % ethanol, 0.05 % Tween20, pH 7.4 (hereafter referred to as incubation buffer) in a total volume of 200 µL/well. Non-specific binding of the radiotracer was determined by coincubation with 400 nM non-labeled compound 1 or compound 2. The optimal fibril concentrations were determined using a concentration determination assay.

Plates covered by removable sealing tape (PerkinElmer) were incubated on a shaker (MaxQ^{™} 6000, orbit diameter 1.9 cm, Thermo Fisher Scientific Inc., Marietta, OH, USA) at 45 rpm for two hours at 37 °C. After incubation, bound and free radioligands were separated by vacuum filtration through glass fiber filtermat B (PerkinElmer) using a filtermat harvester (PerkinElmer). To harvest plates containing αSYN and Aβ₁₋₄₂ fibrils, the filtermat was additionally incubated with 5 mg/mL polyethylenimine for 30 minutes at 4 °C prior to harvesting. The filter was washed three times with 100 mL (approximately 1 mL/well) of ice cold incubation buffer and subsequently dried in a microwave oven for 2.5 minutes at medium power. Melt on scintillator sheets (MeltiLex^{™} B/HS, PerkinElmer) were melted into the filter using a heating plate set to 120 °C. After hardening at room temperature, the filter was sealed in a plastic sample bag (PerkinElmer). Accumulation of tritium was immediately counted in a Wallac MicroBeta^{®} TriLux liquid scintillation counter (PerkinElmer). Radioactivity was plotted against ³H-labeled compound concentration. Data points were fitted using nonlinear regression analysis in GraphPad Prism (GraphPad Software, Inc., version 7.03, La Jolla (CA), USA).

### 3a) Competition binding assay

A fixed concentration of sonicated human recombinant αSYN (200 nM/well) or tau46 (208 nM/well) fibrils diluted in phosphate buffered saline (PBS) were incubated in low binding plates (96 well micro test plate, Ratiolab GmbH, Dreieich, Germany) together with 1 nM [³H]-compound 1 and 1:4 serial dilutions of the cold compound of interest starting from 1 µM diluted in 50 mM Tris-base, 10 % EtOH, 0.05 % Tween20, pH 7.4. For the calculation of Kᵢ values the K_{D} value of compound 1 toward sonicated human recombinant αSYN fibrils was set to 3 nM.

Assay plates were incubated under agitation at 37°C for 2 h covered by plastic foil (Resealable tape, PerkinElmer, Waltham, MA, USA). Prior to harvesting, a filter (Printed filtermat B, PerkinElmer, Waltham, MA, USA) was incubated with 5 mg/mL polyethylenimine (PEI, Poly(ethyleneimine) solution, Sigma Aldrich Chemie GmbH, Taufkirchen, Germany) at 4 °C for 30 min. After incubation, bound and free ligands were separated by vacuum filtration using a harvester (Filtermate harvester, PerkinElmer, Waltham, MA, USA). The filter was washed three times with buffer cooled to 4 °C and subsequently dried in the microwave for 2 min at medium power. The filter was sealed in a plastic bag (Sample bag for MicroBeta^{®}, PerkinElmer, Waltham, MA, USA) together with added scintillator (BETAPLATE SCINT, PerkinElmer, Waltham, MA, USA). Accumulation of tritium was counted in a liquid scintillation counter (Wallac MicroBeta^{®} TriLux, 1450 LSC & Luminescence Counter, PerkinElmer, Waltham, MA, USA) immediately. Radioactivity was plotted against the increasing tritium-labeled compound or cold compound concentrations. Data points were fitted using nonlinear regression analysis in GraphPad Prism (GraphPad Software, Inc., Version 7.03, La Jolla, CA, USA).

### 3b) Modified competition binding assay

For competition binding experiments, a fixed concentration of recombinant αSYN fibrils (sonicated, 15 nM/well) was incubated with 1 nM [³H]-compound 1 or [³H]-compound 2 and decreasing concentrations of a serial dilution of non-labeled competitor (serial dilution 1:4 or 1:3.5 or 1:3 providing the concentration ranges of non-labeled competitor 1 µM-1 pM or 1 µM-4 nM or 1 µM-17 pM, respectively) in 30 mM Tris-HCl, 10 % ethanol, 0.05 % Tween20, pH 7.4 in a total volume of 200 µL/well. Plates were incubated for 4.5 hours at RT covered by removable sealing tape (PerkinElmer, Waltham, MA, USA).

Filtration and readout were performed as described for saturation binding assays. [³H]-labeled ligand binding [in CMP] was plotted against increasing competitor concentrations and data points were fitted using non-linear regression analysis to calculate the IC₅₀ and Kᵢ values (GraphPad Software, Inc., Version 7.03, La Jolla (CA), USA). In the competition binding assay with sonicated recombinant αSYN fibrils, the calculation of K, values was done based on K_{D} values of 0.6 nM and 0.2 nM for compound 1 and compound 2, respectively.

The fibril-binding saturation assay provides K_{D} values. This assay is performed with directly radioactively labeled compounds (e.g. ³H labeling). K_{D} values are shown in Tables 3a and 3b. The saturation binding assays were performed using as target structures fibrillar aggregates produced from recombinant human alpha-synuclein, Aβ₁₋₄₂, and tau46, respectively.

The results show a high affinity for alpha-synuclein fibrils. Compound 1, compound 2, and compound 12 showed a very high affinity with K_{D} values below 10 nM. Compound 1 and compound 2 were also tested in regard to binding to Abeta and tau46 fibrils and showed a good to excellent selectivity, which proves the suitability of these compounds for diagnostic detection of aggregated alpha-synuclein.

**Table 3a: Saturation assay according to protocol 2a**

| Comp. no. | K_{D} [nM] for aggregated alpha-synuclein | K_{D} [nM] for aggregated tau46 | K_{D} [nM] for aggregated Abeta |
|---|---|---|---|
| 1 | < 2 | >5 | > 100 |
| 2 | < 1 | > 15 | > 100 |
| 12 | 7 | N/A | N/A |
| 18 | 126 | N/A | N/A |

**Table 3b: Saturation assay according to protocol 2b**

| Comp. no. | K_{D} [nM] for aggregated alpha-synuclein |
|---|---|
| 1 | 0.6 |
| 2 | 0.2 |

The fibril competition assay provides Kᵢ values. Kᵢ is a quantitative measure that represents the concentration of the non-radioactive tested compound (competitor ligand) required to replace 50% of a reference compound, in this case [³H]-compound 1 (1 nM) or [³H]-compound 2 (1 nM), which is bound to the target structures (1 nM), in our case recombinant alpha-synuclein and tau46 fibrils. This assay is suitable for a screening of non-radioactive ligands. The Kᵢ values obtained for the tested compounds are shown in Tables 4a, 4b and 5.

**Table 4a: Competition assay according to protocol 3a (reference ligand [³H]-compound 1 (1 nM))**

| | Experiment A | Experiment B | |
|---|---|---|---|
| comp. no. | Kᵢ (nM) alpha-synuclein (fibrils) | Kᵢ (nM) alpha-synuclein (sonicated fibrils) | Kᵢ (nM) tau46 (fibrils) |
| 2 | N/A | < 2 | 9 |
| 3 | < 0,1 | 3 | 4 |
| 4 | 8 | 13 | 31 |
| 5 | 27 | >60 | N/A |
| 6 | 24 | >300 | N/A |
| 8 | 43 | N/A | N/A |
| 9 | 2 | < 4 | 28 |
| 10 | 34 | 25 | >100 |
| 12 | 47 | 9 | n.a. |
| 12* | 42 | N/A | N/A |
| 17 | N/A | 2 | 1 |

| | | | |
|---|---|---|---|
| * repetition | | | |

**Table 4b: Competition assay according to protocol 3b (reference ligand [³H]-compound 1 (1nM))**

| comp. no. | Kᵢ (nM) alpha-synuclein (sonicated fibrils) |
|---|---|
| 2 | 0.3 |
| 1 | 0.4 |
| 9 | 2.1 |
| 56 | 2.6 |
| 20 | 3.1 |
| 57 | 3.3 |
| 58 | 5.1 |
| 16 | 5.2 |
| 15 | 6.7 |

**Table 5: Competition assay according to protocol 3b (reference ligand [³H]-compound 2 (1 nM))**

| comp. no. | Structure | Kᵢ (nM) alpha-synuclein (sonicated fibrils) |
|---|---|---|
| **Compounds with non-cyclic amine groups** | | |
| 1 | 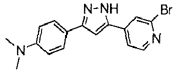 | 0.1 |
| 45 | 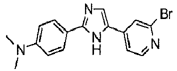 | 0.2 |
| 21 | 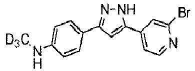 | 0.3 |
| 22 | 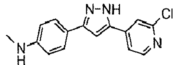 | 0.3 |
| 43 | 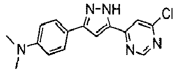 | 0.5 |
| 23 | 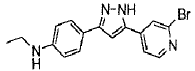 | 0.5 |
| 44 | 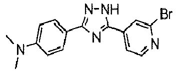 | 0.6 |
| 25 | 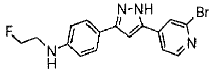 | 0.7 |
| 4 | 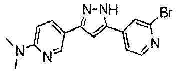 | 0.7 |
| 24 | 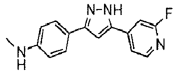 | 0.8 |
| 53 | 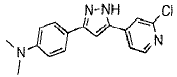 | 0.8 |
| 3 | 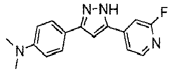 | 0.95 |
| 57 | 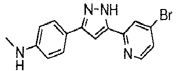 | 2.8 |
| 56 | 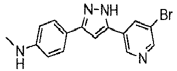 | 3.3 |
| 40 | 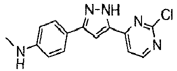 | 3.9 |
| 39 | 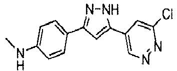 | 4.2 |
| 58 | 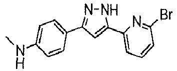 | 5 |
| 6 | 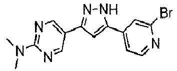 | 8.9 |
| 8 | 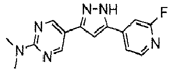 | 9.9 |
| 36 | 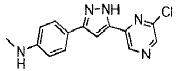 | 12 |
| 5 | 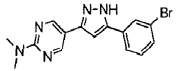 | 13 |
| 31 | 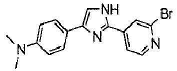 | 13 |
| 41 | 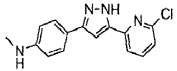 | 14 |
| 37 | 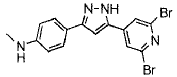 | 16 |
| 42 | 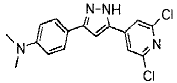 | 23 |
| 38 | 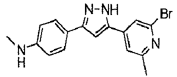 | 113 |

| **Compounds with cyclic amine groups** | | |
|---|---|---|
| 17 | 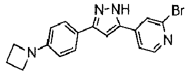 | 0.8 |
| 50 | 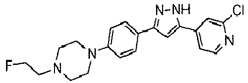 | 0.9 |
| 26 | 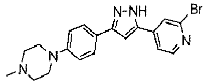 | 1 |
| 55 | 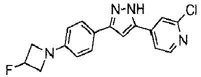 | 1.2 |
| 27 | 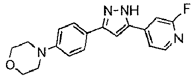 | 1.3 |
| 51 | 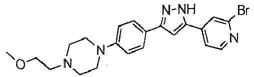 | 2.7 |
| 52 | 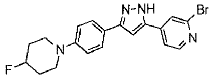 | 3.1 |
| 49 | 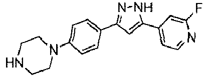 | 61 |
| 35 | 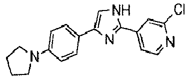 | 168 |

| **Compounds with methylenedioxy groups** | | |
|---|---|---|
| 14 | 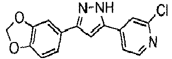 | 1.3 |
| 9 | 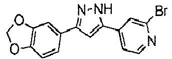 | 3.3 |
| 12 | 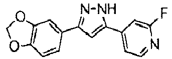 | 3.4 |
| 28 | 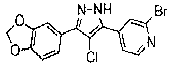 | 4.2 |
| 16 | 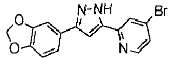 | 4.7 |
| 20 | 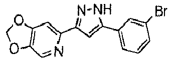 | 5.5 |
| 29 | 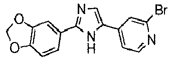 | 6.3 |
| 15 | 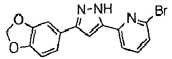 | 6.6 |
| 11 | 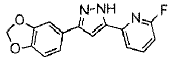 | 7.4 |
| 10 | 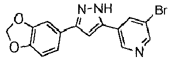 | 7.8 |
| 54 | 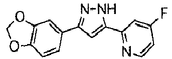 | 8.4 |
| 30 | 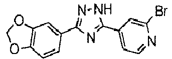 | 12 |
| 32 | 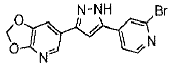 | 18 |
| 33 | 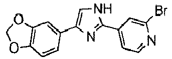 | 33 |
| 34 | 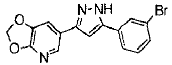 | 41 |
| 48 | 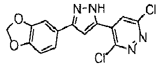 | 59 |
| 47 | 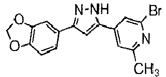 | 97 |
| 13 | 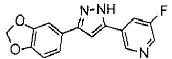 | 189 |

The results of the various competition assays indicate a high binding affinity to alpha-synuclein fibrils for tested compounds (lower Kᵢ values indicating higher binding affinity). In case of competition assay relative to the compound 1 (Table 4a), similar Kᵢ values for alpha-synuclein and tau46 fibrils indicate a similar selectivity as compound 1, for those compounds with higher Kᵢ values for tau46 fibrils than for alpha-synuclein fibrils, the data suggest a further improved selectivity.

Additionally, the compounds 63, 64 and 65 were tested in direct comparison to corresponding compounds bearing the nitrogen atom in one of the phenyl rings. Whereas in Table 6 for compound 1 the Kᵢ value of 0.4 nM was obtained, the Kᵢ value for compound 65 (which lacks the nitrogen atom in the phenyl ring) was much higher (1.9 nM) indicating a significantly improved binding affinity for compound 1. Analysis of the binding properties based on Kᵢ values revealed significantly improved binding affinities for N-containing compounds. The Kᵢ values obtained for the tested compounds in competition binding assay with the reference ligand [³H]-compound 1 (1nM) are shown in Table 6.

**Table 6: Competition assay according to protocol 3b (reference ligand [³H]-compound 1 (1nM))**

| # | Structure | Kᵢ (nM)* |
|---|---|---|
| **Comparison series 1** | | |
| 9 | 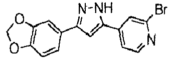 | 2.1 |
| 20 | 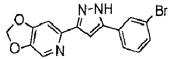 | 3.1 |
| 16 | 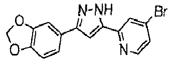 | 5.2 |
| 15 | 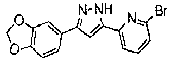 | 6.7 |
| 63 | 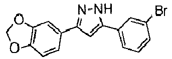 | 9.8 |

| **Comparison series 2** | | |
|---|---|---|
| 2 | 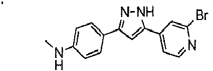 | 0.3 |
| 57 | 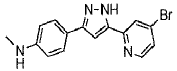 | 3.3 |
| 64 | 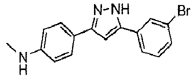 | 3.4 |

| **Comparison series 3** | | |
|---|---|---|
| 1 | 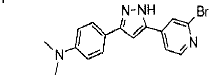 | 0.4 |
| 65 | 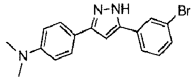 | 1.9 |

| | | |
|---|---|---|
| * sonicated alpha-synuclein fibrils | | |

### Example 3: Therapeutic effect in cell culture

To identify potential therapeutically useful effects, compounds were tested in two cellular models of aggregated alpha-synuclein-dependent toxicity in H4 cells. These cellular models allow for the inducible overexpression of alpha-synuclein hemi-Venus fusion constructs (V1S+SV2) or full-length alpha-synuclein (the model is described in detail in: Bartels M, Weckbecker D, Kuhn PH, Ryazanov S, Leonov A, Griesinger C, Lichtenthaler SF, Bötzel K, Giese A: Iron-mediated aggregation and toxicity in a novel neuronal cell culture model with inducible alpha-synuclein expression, Sci. Rep. 2019 Jun 24; 9(1):9100). Moreover, addition of DMSO and FeCl₃ promotes the aggregation of alpha-synuclein which is associated with an increase in cytotoxicity. The cell culture assay uses these effects by determining the change in the number of cells and the change in the percentage of condensed nuclei, which is indicative of apoptosis, in order to obtain information on which compounds show the most promising effects and thus might be therapeutically useful.

The data is summarized in Table 7 below. In these experiments, cells were incubated with 100 µM FeCl₃ and 0.75% DMSO in the presence of 10 µM anle138c as a positive control, compounds 1 to 19 (10 µM), or DMSO as a negative control. After 48 h, the cells were imaged with an OPERA high-throughput imaging system and analyzed using Acapella software (Perkin Elmer). The table shows the change in number of cells and the change in the fraction of cells with condensed nuclei (i.e. apoptotic cells) relative to the DMSO control. A reduction in the fraction of condensed nuclei (in the absence of a strong reduction in cell number) is indicative of a beneficial therapeutic effect.

**Table 7: Therapeutic compound effects in cellular models**

| | **V1S+SV2 (H4 cells)** | | **alpha-synuclein (H4 cells)** | |
|---|---|---|---|---|
| | **Change in cell number** | **Change in fraction of condensed nuclei** | **Change in cell number** | **Change in fraction of condensed nuclei** |
| Comp. No. | (rel. to DMSO control) [%] | (rel. to DMSO control) [%] | (rel. to DMSO control) [%] | (rel. to DMSO control) [%] |
| 1 | **-21.2** | **-11.7** | **-27.3** | **-65.4** |
| 2 | **-25.8** | **-49.4** | **-44.2** | **-79.0** |
| 3 | **-11.3** | **-31.9** | **-34.8** | **-79.1** |
| 4 | **-36.9** | **33.7** | **-48.6** | **-30.1** |
| 5 | **-12.4** | **-27.3** | **-14.6** | **-34.2** |
| 6 | **-34.0** | **53.6** | **-31.0** | **5.4** |
| 7 | **-30.8** | **-46.1** | **-37.8** | **-62.5** |
| 8 | **-4.5** | **14.2** | **-5.7** | **10.7** |
| 9 | **-22.5** | **-31.8** | **-35.3** | **-23.8** |
| 10 | **-32.8** | **-50.3** | **-35.3** | **-60.2** |
| 11 | **-17.4** | **123.2** | **-20.0** | **13.7** |
| 12 | **-22.2** | **24.9** | **-9.8** | **28.4** |
| 13 | **-6.1** | **46.0** | **-18.1** | **-55.5** |
| 14 | **-21.9** | **61.0** | **-32.0** | **-47.3** |
| 15 | **-11.3** | **23.1** | **-11.2** | **20.3** |
| 16 | **-40.2** | **56.8** | **-47.1** | **0.8** |
| 17 | **-8.2** | **-38.3** | **-28.9** | **-55.0** |
| 18 | **-11.8** | **-19.9** | **-21.3** | **-18.2** |
| 19 | **-5.7** | **3.2** | **-30.3** | **-13.2** |

### Example 4: In vivo biodistribution of [¹¹C]-labeled compound 1 and compound 2

[¹¹C]-labeled compound 1 and compound 2, respectively, were injected intravenously in the tail vein of mice. Mice were then imaged in a small animal PET instrument. For both compounds, a good blood-brain-barrier penetration with SUV values > 1.5 was observed. In addition, a rapid washout from the brain was found. Clearance half-life for [¹¹C]-labeled compound 1 and compound 2 was 12 minutes and 9 minutes, respectively.

### Example 5: Autoradiography using human brain tissue

Autoradiography was performed on histological slices of frozen brain tissue using tritiated compound 1 and brain tissue derived from the cingulate gyrus of a patient with dementia with Lewy bodies. When using [³H]-compound 1 at a concentration 3 nM for incubation of the tissue followed by washing steps, a preferential binding to the gray matter can be observed which is a pattern that is identical to the known distribution of aggregated alpha-synuclein (Figure 1, left panel). When specific binding is blocked by an excess of non-tritiated (i.e. "cold") compound 1, no to very low unspecific binding of compound 1 to brain tissue can be seen (Figure 1, right panel).

These findings indicate that compound 1 specifically and with high affinity binds to pathologically aggregated alpha-synuclein present in a human synucleinopathy patient and allows diagnostic detection of aggregated alpha-synuclein.

The present invention is characterized by the following preferred embodiments:
1. A compound represented by the general formula Ia, Ib, Ila or lib 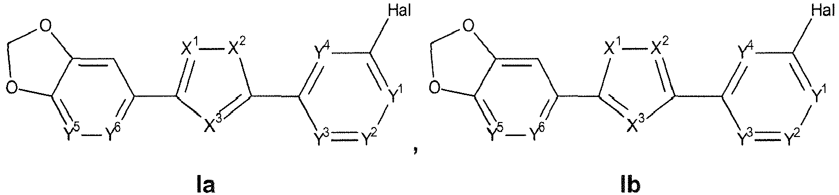 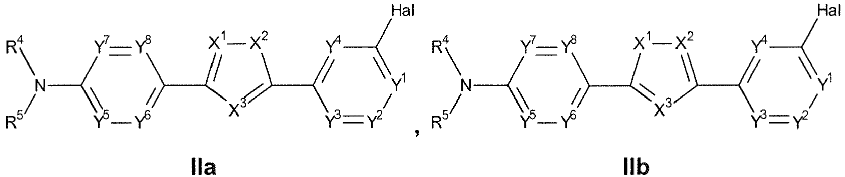 or a prodrug, solvate or salt thereof,
   wherein
   **X¹, X²,** and **X³** are independently selected from CR², N and NR¹, with the proviso that at least two of X¹, X², and X³ are N or NR¹;
   **Y¹, Y², Y³, Y⁴, Y⁵, Y⁶, Y⁷** and **Y⁸** are independently selected from CR³ and N, with the proviso that at least one of Y¹, Y², Y³, Y⁴, Y⁵, Y⁶, Y⁷ and Y⁸ is N;
   **R¹** is independently selected from hydrogen, C₁₋₄ alkyl and -(CH₂)-O-P(=O)(OR)(OR), wherein C₁₋₄ alkyl can be optionally substituted by one or more halogen;
   **R²** is independently selected from hydrogen, halogen, and C₁₋₄ alkyl, wherein C₁₋₄ alkyl can be optionally substituted by one or more halogen;
   **R** is hydrogen or a cation;
   **R³** is hydrogen, halogen, C₁₋₄ alkyl, OH and C₁₋₄ alkoxy, wherein C₁₋₄ alkyl and C₁₋₄ alkoxy can be optionally substituted by one or more halogen;
   **R⁴** and **R⁵** are independently selected from H and C₁₋₄ alkyl, wherein C₁₋₄ alkyl can be optionally substituted by one or more halogen or wherein R⁴ and R⁵ together with the nitrogen atom to which they are bound form a 4- to 6-membered saturated, heterocyclic ring which optionally contains one or more heteroatoms selected from O and N in addition to the nitrogen atom to which R⁴ and R⁵ are bound, wherein the 4- to 6-membered saturated, heterocyclic ring can be optionally substituted by one or more R⁶;
   **R⁶** is independently selected from halogen, C₁₋₄ alkyl, OH and C₁₋₄ alkoxy, wherein C₁₋₄ alkyl and C₁₋₄ alkoxy can be optionally substituted by one or more halogen; and
   **Hal** is halogen.
2. The compound according to item 1, wherein 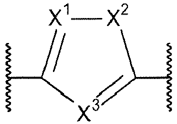 is 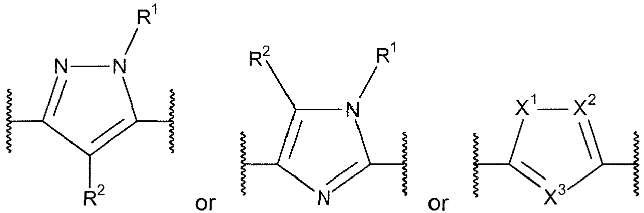 is 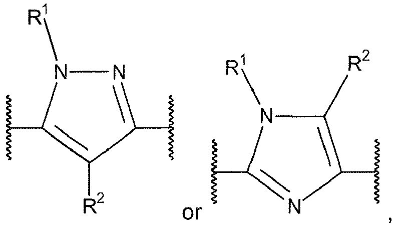 wherein R¹ and R² are as defined in item 1.
3. The compound according to item 2, wherein 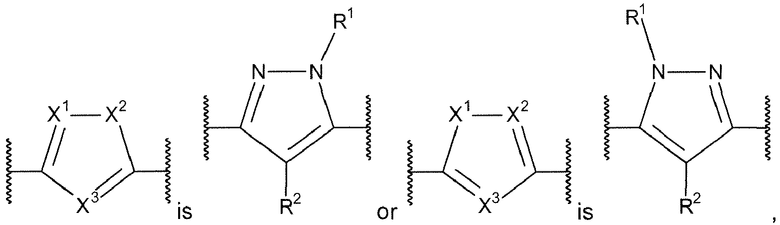 wherein R¹ and R² are as defined in item 1.
4. The compound according to any one of items 1 to 3, wherein one or two or three of Y¹, Y², Y³, Y⁴, Y⁵, Y⁶, Y⁷ and Y⁸ is/are N.
5. The compound according to any one of items 1 to 4, wherein
   in the formulae Ia or Ib, Y¹ is N and at least one of Y³, Y⁴, and Y⁶ is N; or
   in the formulae Ila or lib, Y¹ is N and at least one of Y³, Y⁴, Y⁵, Y⁶, Y⁷ and Y⁸ is N.
6. The compound according to any one of items 1 to 5, wherein Y¹ is N and Y², Y³, Y⁴, Y⁵, Y⁶, Y⁷ and Y⁸ are CR³, preferably Y¹ is N and Y², Y³, Y⁴, Y⁵, Y⁶, Y⁷ and Y⁸ are CH.
7. The compound according to any one of items 1 to 6, wherein R² is H.
8. The compound according to any one of items 1 to 7, wherein at least one of R⁴ and R⁵ is C₁₋₄ alkyl, wherein C₁₋₄ alkyl can be optionally substituted by one or more halogen.
9. The compound according to any one of items 1 to 7, wherein R⁴ and R⁵ together with the nitrogen atom to which they are bound form a 4- to 6-membered saturated, heterocyclic ring which optionally contains one or more heteroatoms selected from O and N in addition to the nitrogen atom to which R⁴ and R⁵ are bound, wherein the 4- to 6-membered saturated, heterocyclic ring can be optionally substituted by one or more R⁶.
10. The compound according to item 9, wherein the 4- to 6-membered saturated, heterocyclic ring is selected from azetidinyl, pyrrolidinyl, piperidinyl, morpholinyl and piperazinyl, wherein azetidinyl, pyrrolidinyl, piperidinyl, morpholinyl and piperazinyl can be optionally substituted by one or more R⁶.
11. The compound according to any one of items 1 to 10 or a prodrug, solvate or salt thereof, wherein the compound is detectably labeled, preferably wherein the compound is detectably labeled by ¹⁸F, ¹¹C, ¹²⁵I, ¹²³I, ¹³¹I, ⁷⁷Br and ⁷⁶Br, more preferably wherein the compound is detectably labeled by.¹⁸F or ¹¹C.
12. A diagnostic composition comprising a compound as defined in any one of items 1 to 11 or a solvate or salt thereof and optionally a pharmaceutically acceptable carrier.
13. A pharmaceutical composition comprising a compound as defined in any one of items 1 to 10 or a prodrug, solvate or salt thereof and optionally a pharmaceutically acceptable carrier.
14. A compound according to any one of items 1 to 11 or a prodrug, solvate or salt thereof for use in the diagnosis of a disease linked to alpha-synuclein aggregation.
15. A compound according to any one of items 1 to 10 or a prodrug, solvate or salt thereof for use in the treatment of a disease linked to alpha-synuclein aggregation.
16. The compound for use according to item 14 or 15, wherein the disease linked to alpha-synuclein aggregation is selected from Parkinson's disease, dementia with Lewy bodies, and multiple system atrophy.
17. Use of a compound according to any one of items 1 to 11 or a prodrug, solvate or salt thereof for inhibiting alpha-synuclein aggregation *in vitro* or *ex vivo.*
18. A compound according to any one of items 1 to 11 or a solvate or salt thereof for use in imaging alpha-synuclein aggregates.
19. A method of imaging deposits of aggregated alpha-synuclein, the method comprising the steps of:
   (i) introducing a detectable quantity of a detectably labeled compound according to claim 11 into a subject;
   (ii) allowing sufficient time for the compound to be associated with the aggregated alpha-synuclein; and
   (iii) detecting the compound associated with the aggregated alpha-synuclein.
20. A kit for preparing a detectably labeled compound according to item 11 or a solvate or salt thereof, wherein the kit comprises at least two precursor compounds which upon reaction form the compound as defined in item 11 or a solvate or salt thereof.

## Claims

1. A compound represented by the general formula IIa or IIb 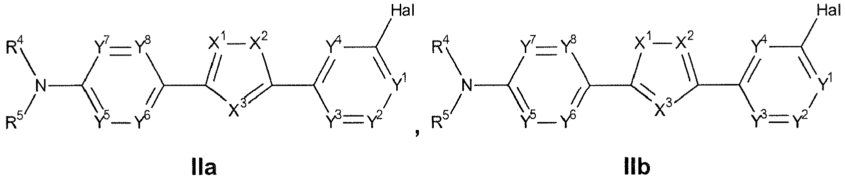 or a solvate or salt thereof,
wherein 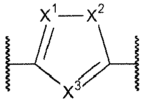 is selected from 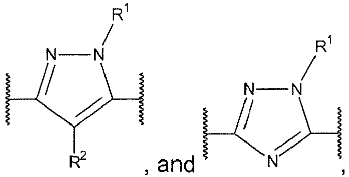 and 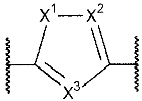 is selected from 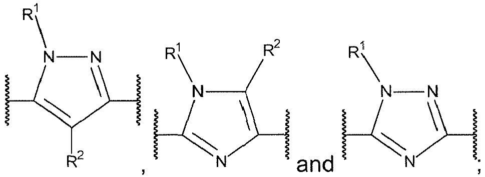
Y¹, Y², Y³, Y⁴, Y⁵, Y⁶, Y⁷ and Y⁸ are independently selected from CR³ and N, with the proviso that at least one of Y¹, Y², Y³, Y⁴, Y⁵, Y⁶, Y⁷ and Y⁸ is N and with the proviso that in the formulae la or lb, at least one of Y¹, Y³, Y⁴, and Y⁶ is N;
**R¹** is independently selected from hydrogen, C₁₋₄ alkyl and -(CH₂)-O-P(=O)(OR)(OR), wherein C₁₋₄ alkyl can be optionally substituted by one or more halogen;
**R²** is independently selected from hydrogen, halogen, and C₁₋₄ alkyl, wherein C₁₋₄ alkyl can be optionally substituted by one or more halogen;
**R** is hydrogen or a cation;
**R³** is hydrogen;
**R**⁴ and **R**⁵ are independently selected from H and C₁₋₄ alkyl, wherein C₁₋₄ alkyl can be optionally substituted by one or more halogen or wherein R⁴ and R⁵ together with the nitrogen atom to which they are bound form a piperazinyl ring which is substituted by one or more R⁶; and
**R⁶** is independently selected from halogen, C₁₋₄ alkyl, OH and C₁₋₄ alkoxy, wherein C₁₋₄ alkyl and C₁₋₄ alkoxy can be optionally substituted by one or more halogen; and **Hal** is halogen.

2. The compound according to claim 1, wherein 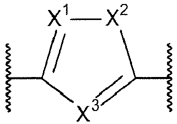 is 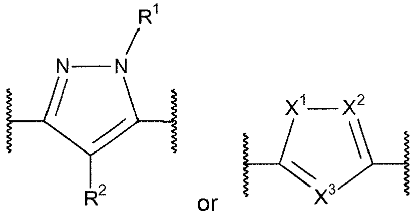 is 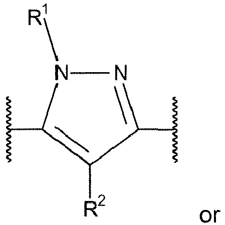 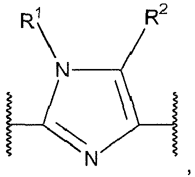 wherein R¹ and R² are as defined in claim 1.

3. The compound according to claim 2, wherein 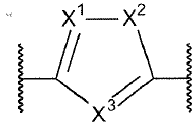 is 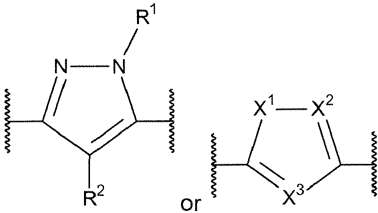 is 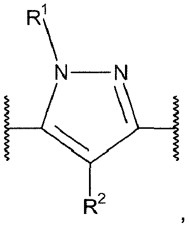 wherein R¹ and R² are as defined in claim 1.

4. The compound according to any one of claims 1 to 3, wherein one or two or three of Y¹, Y², Y³, Y⁴, Y⁵, Y⁶, Y⁷ and Y⁸ is/are N.

5. The compound according to any one of claims 1 to 4, wherein
in the formulae la or lb, Y¹ is N and at least one of Y³, Y⁴, and Y⁶ is N; or
in the formulae Ila or lib, Y¹ is N and at least one of Y³, Y⁴, Y⁵, Y⁶, Y⁷ and Y⁸ is N.

6. The compound according to any one of claims 1 to 5, wherein Y¹ is N and Y², Y³, Y⁴, Y⁵, Y⁶, Y⁷ and Y⁸ are CR³, preferably Y¹ is N and Y², Y³, Y⁴, Y⁵, Y⁶, Y⁷ and Y⁸ are CH.

7. The compound according to any one of claims 1 to 6, wherein R² is H.

8. The compound according to any one of claims 1 to 7 or a solvate or salt thereof, wherein the compound is detectably labeled, preferably wherein the compound is detectably labeled by ¹⁸F, ¹¹C, ¹²⁵I, ¹²³I, ¹³¹I, ⁷⁷Br and ⁷⁶Br, more preferably wherein the compound is detectably labeled by^{,18}F or ¹¹C.

9. A diagnostic composition comprising a compound as defined in any one of claims 1 to 8 or a solvate or salt thereof and optionally a pharmaceutically acceptable carrier.

10. A pharmaceutical composition comprising a compound as defined in any one of claims 1 to 7 or a solvate or salt thereof and optionally a pharmaceutically acceptable carrier.

11. A compound according to any one of claims 1 to 8 or a solvate or salt thereof for use in the diagnosis of a disease linked to alpha-synuclein aggregation.

12. A compound according to any one of claims 1 to 7 or a solvate or salt thereof for use in the treatment of a disease linked to alpha-synuclein aggregation.

13. The compound for use according to claim 11 or 12, wherein the disease linked to alpha-synuclein aggregation is selected from Parkinson's disease, dementia with Lewy bodies, and multiple system atrophy.

14. Use of a compound according to any one of claims 1 to 8 or a solvate or salt thereof for inhibiting alpha-synuclein aggregation *in vitro* or ex *vivo.*

15. A compound according to any one of claims 1 to 8 or a solvate or salt thereof for use in imaging alpha-synuclein aggregates.

16. A method of imaging deposits of aggregated alpha-synuclein, the method comprising the steps of:
(i) introducing a detectable quantity of a detectably labeled compound according to claim 8 into a subject;
(ii) allowing sufficient time for the compound to be associated with the aggregated alpha-synuclein; and
(iii) detecting the compound associated with the aggregated alpha-synuclein.

17. A kit for preparing a detectably labeled compound according to claim 8 or a solvate or salt thereof, wherein the kit comprises at least two precursor compounds which upon reaction form the compound as defined in claim 8 or a solvate or salt thereof.
